# EUROPÄISCHE PATENTSCHRIFT

(11) **EP 1 025 100 B1**
(45) Veröffentlichungstag und Bekanntmachung des Hinweises auf die Patenterteilung: **23.01.2002**
(21) Anmeldenummer: 98951491.4
(22) Anmeldetag: 05.10.1998
(51) Int. Cl.: C07D 471/00

(54) **3-SUBSTITUIERTE TETRAHYDROPYRIDOPYRIMIDINON-DERIVATE, IHRE HERSTELLUNG UND VERWENDUNG**
3-SUBSTITUTED TETRAHYDROPYRIDOPYRIMIDINONE DERIVATIVES, METHOD FOR PRODUCING THE SAME, AND THEIR USE
DERIVES DE TETRAHYDROPYRIDOPYRIMIDINONE SUBSTITUES EN POSITION 3, LEUR PRODUCTION ET LEUR UTILISATION

(30) Priorität: 24.10.1997 DE 19747063
(43) Veröffentlichungstag der Anmeldung: 09.08.2000
(73) Patentinhaber: BASF AKTIENGESELLSCHAFT, 67056 Ludwigshafen (DE)
(72) Erfinder: LUBISCH, Wilfried, D-68159 Mannheim (DE); DULLWEBER, Uta, D-67227 Frankenthal (DE); STARCK, Dorothea, D-67059 Ludwigshafen (DE); STEINER, Gerd, D-67281 Kirchheim (DE); BACH, Alfred, D-69123 Heidelberg (DE); EMLING, Franz, D-67065 Ludwigshafen (DE); GARCIA-LADONA, Xavier, D-76870 Kandel (DE); TESCHENDORF, Hans-Jürgen, D-67373 Dudenhofen (DE); WICKE, Karsten, D-67112 Altrip (DE)
(74) Vertreter: Grünecker, Kinkeldey, Stockmair & Schwanhäusser Anwaltssozietät
(86) Internationale Anmeldenummer: EP9806305
(87) Internationale Veröffentlichungsnummer: WO9921857

(56) Entgegenhaltungen:
- F. CLAUDI ET AL.: "3-[2-(4-Fluorobenzoyl)piperidin-1-yl]ethy l]-5,6,7,8-tetrahydro-4(3H)-quinazolinones : serotonin 5-HT2A receptor antagonists endowed with potent central action" EUROPEAN JOURNAL OF MEDICINAL CHEMISTRY.CHIMICA THERAPEUTICA., Bd. 32, Nr. 7-8, 1997, Seiten 651-658, XP002095654 PARIS FR

## Beschreibung

Die Erfindung betrifft 3-substituierte Tetrahydropyridopyrimidinon-Derivate, deren Herstellung und Verwendung zur Herstellung von Pharmawirkstoffen.

Die klassischen Antidepressiva und auch die neueren selektiven Serotonin Reuptakehemmer (SSRIs) entfalten ihre antidepressive Wirkung unter anderem durch die Inhibierung der aktiven Wiederaufnahme des Transmitters in die präsynaptischen Nervenendigungen. Leider tritt dabei die antidepressive Wirkung erst nach einer Behandlung von mindestens 3 Wochen ein, zudem sind ca. 30 % der Patienten therapieresistent.

Die Blockade von präsynaptischen Serotonin-Autorezeptoren erhöht durch Aufhebung der negativen Kopplung die Serotoninfreisetzung und damit die aktuelle Transmitterkonzentration im synaptischen Spalt. Dieser Anstieg der Transmitterkonzentration gilt als das antidepressive Wirkprinzip. Dieser Wirkmechanismus unterscheidet sich von den bisher bekannten Antidepressiva, die zugleich die präsynaptischen und somatodendritischen Autorezeptoren aktivieren und deshalb erst nach Desensibilisierung dieser Autorezeptoren zum verzögerten Wirkungseintritt führen. Die direkte Autorezeptor-Blockade umgeht diesen Effekt.

Die in JP 08027149 und JP 04054181 beschriebenen Derivate sind bekannt.

Nach bisherigem Wissen handelt es sich bei dem präsynaptischen Serotonin-Autorezeptor um den 5-HT_{1B}-Subtyp (Fink et al., Arch. Pharmacol. 352 (1995), S. 451). Dessen selektive Blockade durch 5-HT_{1B/D}-Antagonisten erhöht die Serotonin-Freisetzung im Gehirn: G.W. Price et al., Behavioural Brain Research 73 (1996), S. 79-82; P.H. Hutson et al., Neuropharmacology Vol. 34, No. 4 (1995), S. 383-392.

Der selektive 5-HT_{1B}-Antagonist GR 127 935 vermindert jedoch überraschenderweise die Serotonin-Freisetzung im Cortex nach systemischer Gabe. Eine Erklärung könnte die Stimulierung von somatodendritischen 5-HT_{1A}-Rezeptoren in der Raphe Region durch das freigesetzte Serotonin sein, die die Feuerrate serotonerger Neuronen und damit die Serotonin-Ausschüttung hemmt (M. Skingle et al., Neuropharmacology Vol. 34 No. 4 (1995), S. 377-382, S. 393-402).

Eine Strategie zur Umgehung der autoinhibitorischen Effekte in serotonergen Ursprungsgebieten verfolgt also die Blockade der präsynaptischen 5-HT_{1B} Rezeptoren. Diese Hypothese wird gestützt durch die Beobachtung, daß der Einfluß von Paroxetine auf die Serotonin-Freisetzung im dorsalen Raphe Nucleus der Ratte durch den 5-HT_{1B}-Rezeptor Antagonisten GR 127 935 potenziert wird (Davidson and Stamford, Neuroscience Letts., 188 (1995), 41).

Die zweite Strategie schließt die Blockade beider Typen von Autorezeptoren mit ein, nämlich die 5-HT_{1A}-Rezeptoren, um das neuronale Feuern zu verstärken, und die 5-HT_{1B}-Rezeptoren, um die terminale Serotonin Freisetzung anzuheben (Starkey and Skingle, Neuropharmacology 33 (3-4) (1994), 393).

5-HT_{1B/D}-Antagonisten allein oder gekoppelt mit einer 5-HT_{1A}-Rezeptor antagonistischen Komponente sollten deshalb vermehrt die Serotonin-Freisetzung im Gehirn erhöhen und könnten deshalb Vorteile in der Therapie von Depressionen und verwandten psychischen Krankheiten beinhalten.

Es wurde nun gefunden, daß 3-substituierte Tetrahydropyridopyrimidinon-Derivate der Formel I 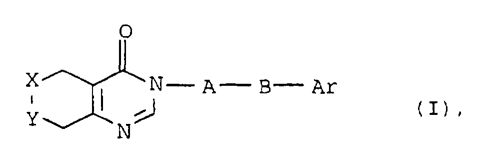 worin
einer der beiden Reste X, Y für CH₂ und der andere für NR¹ steht,
- R¹: für Wasserstoff, (C₁₋₆) Alkyl verzweigt oder unverzweigt, CO-(C₁₋₄)-Alkyl, CO₂tBu, CO-Aryl und einen Phenylalkyl-C₁-C₄-Rest, der seinerseits am Aromaten durch F, Cl, Br, I, C₁-C₄ Alkyl, C₁-C₄ Alkoxy, Trifluormethyl, Hydroxy, Amino, Cyano oder Nitro substituiert sein kann, steht,
- A: für verzweigtes oder unverzweigtes (C₁₋₁₀)-Alkylen oder geradkettiges oder verzweigtes (C₂₋₁₀)-Alkylen steht, das wenigstens eine Gruppe Z umfaßt, die ausgewählt ist unter O, S, NR², Cyclopropyl, CHOH, einer Doppel- oder einer Dreifachbindung,
- R²: für Wasserstoff und C₁-C₄ Alkyl steht,
- B: für 4-Piperidin, 4-Tetrahydro-1,2,3,6 pyridin, 4-Piperazin und die entsprechenden um eine Methylengruppe vergrößerten Ringverbindungen steht, wobei die Verknüpfung zu A über ein N-Atom von B erfolgt und
- Ar: für Phenyl, das gegebenenfalls durch (C₁₋₆) Alkyl verzweigt oder unverzweigt, O-( C₁₋₆)-Alkyl verzweigt oder unverzweigt, OH, F, Cl, Br, I, Trifluormethyl, NR²₂, CO₂R², Cyano oder Phenyl substituiert ist, Tetralin, Indan, höherkondensierte Aromaten wie Naphthalin, das gegebenenfalls durch (C₁₋₄)-Alkyl oder O(C₁₋₆)-Alkyl substituiert ist, Anthracen oder 5- oder 6-gliedrige aromatische Heterocyclen mit 1 bis 2 Heteroatomen, die unabhängig voneinander ausgewählt sind unter O und N, die noch mit weiteren aromatischen Resten anelliert sein können, steht,
sowie deren physiologisch verträgliche Salze wertvolle pharmakologische Eigenschaften besitzen.

Bevorzugt sind insbesondere Verbindungen, in denen
einer der beiden Reste X, Y für CH₂ und der andere für NR¹ steht,
- R¹: für Wasserstoff, (C₁₋₄)-Alkyl verzweigt oder unverzweigt, CO-(C₁₋₄)-Alkyl, CO₂tBu, COPh oder einen Phenylalkyl C₁-C₂-Rest, der seinerseits am Aromaten durch F, Cl, Br, I, C₁-C₄-Alkyl, C₁-C₄-Alkoxy, Trifluormethyl, Hydroxy oder Cyano substituiert sein kann, steht,
- A: für (C₂₋₅) Alkylen verzweigt oder unverzweigt oder eine (C₂₋₅)-Alkylengruppe, die eine Gruppe Z umfaßt, die ausgewählt ist unter CHOH, Cyclopropyl, einer Doppel- oder einer Dreifachbindung,
- B: für 4-Piperidin, 4-Tetrahydro-1,2,3,6 pyridin, 4-Piperazin oder Homopiperazin steht, wobei die Verknüpfung zu A über ein N-Atom von B erfolgt und
- Ar: für Phenyl, das gegebenenfalls durch (C₁₋₆)-Alkyl verzweigt oder unverzweigt, O-( C₁₋₆)-Alkyl verzweigt oder unverzweigt, F, Cl, Br, I, Trifluormethyl, CO₂R², NR²₂, Cyano oder Phenyl substituiert ist, Tetralin, Indan, höherkondensierte Aromaten wie Naphthalin, das gegebenenfalls durch (C₁₋₄) Alkyl oder O(C₁₋₄) Alkyl substituiert ist oder 5- oder 6-gliedrige aromatische Heterocyclen mit 1 bis 2 Stickstoffen, die mit weiteren aromatischen Resten anelliert sein können, steht.

Besonders bevorzugte Verbindungen der Formel I sind solche, bei denen einer der beiden Reste X, Y für CH₂ und der andere für NR¹ steht,
- R¹: für Wasserstoff, (C₁₋₂) Alkyl, CO-( C₁₋₄)-Alkyl oder einen Phenylalkyl C₁-C₂ Rest steht,
A für (C₂₋₃) Alkyl steht,
B für 4-Piperidin, 4-Piperazin oder 4-Tetrahydro-1,2,3,6 pyridin steht und
Ar für Pyrimidin, Phenyl, das gegebenenfalls durch O(C₁₋₂) Alkyl in ortho-Position substituiert ist, Tetralin, Indan oder Naphthalin, das gegebenenfalls durch (C₁₋₄) Alkyl oder O(C₁₋₂) Alkyl substituiert ist, steht.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Die erfindungsgemäßen Verbindungen der Formel I lassen sich herstellen, indem man eine Verbindung der Formel II, 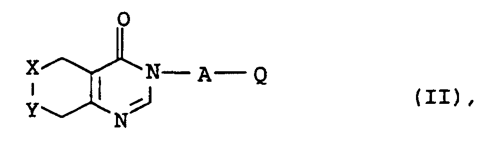 in der A, X und Y die oben angegebene Bedeutung haben und Q für eine abspaltbare Gruppe (z. B. Cl, Br, I, Alkansulfonyloxy oder Arylsulfonyloxy) steht, mit einer Verbindung der Formel III,

H-B-Ar (III),

worin B und Ar die oben angegebene Bedeutung haben, in an sich bekannter Weise umsetzt und die so erhaltene Verbindung gegebenenfalls in das Säureadditionssalz einer physiologisch verträglichen Säure überführt. Es ist ebenfalls möglich, eine Verbindung der Formel IV 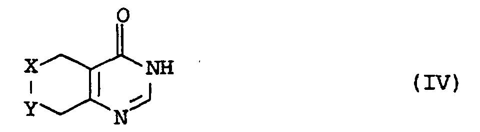 mit einer Verbindung der Formel V

Q-A-B-Ar (V)

in an sich bekannter Weise umzusetzen.

Eine weitere Synthesevariante stellt die Verknüpfung einer Verbindung der Formel VI 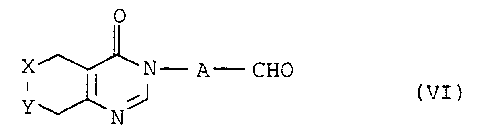 mit einer Verbindung der Formel III durch eine an sich bekannte reduktive Aminierung dar.

Die Synthese von Verbindungen der Formel III kann erfolgen durch
1. Verknüpfung von Verbindungen der allgemeinen Formel VII

   W-B¹ (VII)

   wobei B¹ für Piperazin oder Homopiperazin und W für Wasserstoff oder eine der üblichen Aminoschutzgruppen (wie z.B. Boc oder Cbz) steht, mit einer Verbindung der allgemeinen Formel VIII

   P-Ar (VIII),

   wobei P für B(OH)₂, SnR₃, OTf, Br, Cl, oder I und R für C₁-C₄-Alkyl steht, in bekannter Weise umsetzt; oder
2. die Verknüpfung von Verbindungen der allgemeinen Formel IX

   W-B²-P¹ (IX),

   wobei B¹ für 4-Tetrahydro-1,2,3,6-pyridin und die entsprechenden um eine Methylengruppe vergrößerten Ringverbindungen und P¹ für Cl, Br, I, SnR₃ - wobei R für C₁-C₄-Alkyl steht - OTf steht mit einer Verbindung der allgemeinen Formel X

   P-Ar (X),

   wobei W, P und Ar jeweils die oben genannte Bedeutung besitzen und die Umsetzungen nach bekannten Verfahren erfolgen, wie z.B. beschrieben in
   S.L. Buchwald et al. *J. Am. Chem. Soc.* **1996**, *118,* 7215,
   J.F. Hartwig et al. *Tetrahedron Lett.* **1995**, *36,* 3604,
   J.K. Stille et al. *Angew. Chem.* **1986**, 98, 504,
   S.L. Buchwald et al. *Angew. Chem.* **1995**, *107,* 1456 oder
   J.F. Hartwig et al. *J.Am. Chem. Soc* **1996**, *118,* 7217 oder
   J.F. Hartwig et al. J. *Org. Chem.* **1997**, 62, 1268,
   S.L. Buchwald et al. *J. Org. Chem.* **1997**, 62, 1264 und dort zitierte Literatur oder
   S.L. Buchwald et al *J.Am. Chem. Soc* **1997**, *119*, 6054,
   J.K. Stille, *Angew. Chem.* **1986**, 98, 504 oder
   J.K.Stille et al. *J. Org. Chem.* **1990**, *55,* 3014,
   M. Pereyre et al. "Tin in Organic Synthesis", Butterworth 1987; oder
3. Reduktion von Verbindungen der allgemeinen Formel(XI)

   W-B²-Ar (XI),

   wobei B² die oben angegebene Bedeutung besitzt zu Verbindungen der allgemeinen Formel XII

   W-B³-Ar (XII),

   worin B³ für in 1,4-Stellung verknüpfte Piperidine und die entsprechenden um eine Methylengruppe vergrößerten Ringverbindungen steht; oder
4. die Cyclisierung von Verbindungen der allgemeinen Formel XIII

   W-N-(C₃H₄Q)₂ (XIII),
wobei W und Q die oben beschriebene Bedeutung besitzen, mit einer Verbindung der allgemeinen Formel XIV

NH₂-Ar (XIV),

wobei Ar die oben genannte Bedeutung besitzt, zu Verbindungen der allgemeinen Formel XV

W-B¹-Ar (XV),

Die als Ausgangsstoffe für die Synthese der neuen Verbindungen benötigten Substanzen der Formeln III und V sind bekannt oder lassen sich gemäß den bekannten Verfahren (z. B. *Organikum* Barth Dt. Ver1. der Wiss. **1993** oder A. R. Katritzky, C. W. Rees (ed.) *Comprehensive Heterocyclic Chemistry* Pergamon Press) aus analogen Edukten synthetisieren.

Die weitere Umsetzung der so nach 1. bis 4. mit anschließender Abspaltung etwaiger Schutzgruppen hergestellten Verbindungen

H-B-Ar (III)

zu den Verbindungen der Formel V erfolgt durch Verknüpfung mit Verbindungen der Formel XVI .

Q-A-Q' (XVI),

wobei Q und Q' für Abgangsgruppen stehen, unter an sich bekannten Bedingungen.

Die als Ausgangsstoffe für die Synthese der neuen Verbindungen benötigten Substanzen der Formel II, IV, VI und der allgemeinen Formel P-Ar, NH₂-Ar, W-B¹ bzw. W-B²-P¹ sind bekannt oder lassen sich gemäß. den in der Literatur beschriebenen Herstellverfahren aus analogen Edukten synthetisieren (z.B. B. Dumaitre, N. Dodic *J. Med. Chem.* **1996**, *39*, 1635 oder A. Yokoo et al. *Bull. Chem. Soc. Jpn.* **1956**, 29, 631 oder L. Börjeson et al. *Acta Chem. Chem.* **1991**, 45, 621 oder *Organikum* Barth Dt. Ver1. der Wiss. **1993** oder A. R. Katritzky, C. W. Rees (ed.) *Comprehensive Heterocyclic Chemistry* Pergamon Press oder *The Chemistry of Heterocyclic Compounds* J. Wiley & Sons Inc. NY und der dort jeweils zitierten Literatur).

Die oben beschriebenen Umsetzungen erfolgen im allgemeinen in einem inerten organischen Lösungsmittel, z. B. Dimethylformamid, Acetonitril, Dichlormethan, Dimethylsulfoxid, Dimethoxyethan, Toluol, Ethylacetat, Xylol, einem Keton, wie z.B. Aceton oder Methylethylketon, einem Alkohol, wie beispielsweise Ethanol oder n-Butanol, oder einem cyclischen gesättigten Ether, z.B. Tetrahydrofuran oder Dioxan.

Die Umsetzungen erfolgen in der Regel bei Temperaturen zwischen 20°C und der Siedetemperatur des Lösungsmittels und sind im allgemeinen innerhalb von 1 bis 20 Stunden beendet. Gewünschtenfalls arbeitet man in Gegenwart eines säurebindenden Mittels wie Natrium- oder Kaliumcarbonat, Natriummethylat, Natriumethylat, Natriumhydrid, metallorganischen Verbindungen (Butyllithium, Alkylmagnesiumverbindungen), Kalium-t-butylat, Pyridin oder Triethylamin.

Die Umsetzungen erfolgen gegebenenfalls unter Verwendung eines Katalysators, wie z. B. Übergangsmetalle und deren Komplexe, z.B. Pd-C, Pd(PPh₃)₄, Pd(OAc)₃, Pd(P(oTol)₃)₄, Pd₂(dba)₃ oder Ni(COD)₂.

Die Isolierung des Rohproduktes erfolgt in üblicher Weise, beispielsweise durch Filtration, Abdestillieren des Lösungsmittels oder Extraktion aus dem Reaktionsgemisch.

Die erfindungsgemäßen Verbindungen der Formel I können entweder durch Umkristallisation aus den üblichen organischen Lösungsmitteln oder durch Säulenchromatographie gereinigt werden.

Die Erfindung umfaßt neben den 3-substituierten Tetrahydro-pyridopyrimidinon-Derivaten auch die Säureadditionssalze der Verbindungen der Formel I mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in "Fortschritte der Arzneimittelforschung", Band 10, Seiten 224 ff., Birkhäuser Verlag, Basel und Stuttgart, 1966, beschrieben.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays: Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die erfindungsgemäßen Verbindungen weisen eine hohe Affinität zu den Serotoninrezeptoren 5-HT_{1B}, 5-HT_{1D} und 5-HT_{1A} auf. Die Affinität zu diesen Rezeptoren ist dabei etwa gleich groß, zumindest in der gleichen Größenordnung. Darüberhinaus weisen einige der erfindungsgemäßen Verbindungen eine gute Serotonin-Reuptake Hemmung auf- ein Prinzip, das bei den meisten Antidepressiva verwirklicht ist.

Diese Verbindungen eignen sich als Arzneimittel zur Behandlung von Krankheitszuständen, bei denen die Serotoninkonzentration erniedrigt ist, und bei denen man im Rahmen einer Therapie gezielt die Aktivität der präsynaptischen Rezeptoren 5-HT_{1B}, 5-HT_{1A}, 5-HT_{1D} blockieren möchte, ohne dabei andere Rezeptoren stark zu beeinflussen. Solch ein Krankheitszustand ist beispielsweise die Depression.

Die Verbindungen der vorliegenden Erfindung können auch für die Behandlung von zentralnervös bedingten Gemütsstörungen wie saisonale affektive Störungen und Dysthymie von Nutzen sein. Dazu gehören auch Angstzustände wie generalisierte Angst, Panikanfälle, Soziophobie, Zwangsneurosen und post-traumatische Stress-Symptome, Gedächtnisstörungen einschließlich Demenz, Amnesien und altersbedingter Gedächtnisschwund sowie psychogene Eßstörungen wie Anorexia nervosa und Bulimia nervosa.

Die erfindungsgemäßen Verbindungen können außerdem für die Behandlung endokriner Erkrankungen wie Hyperprolaktinämie sowie für die Behandlung von Gefäßspasmen (insbesondere der Hirngefäße), Hypertonie und gastrointestinalen Störungen, die mit Motilitäts- und Sekretionsstörungen einhergehen, von Nutzen sein. Ein weiteres Anwendungsgebiet sind Sexualstörungen.

Die folgenden Beispiele dienen zur Erläuterung der Erfindung ohne sie zu begrenzen.

### Beispiel 1:

### 3-[2-[4-(2-methoxyphenyl)-1-piperazinyl]ethyl]-3,5,7,8-tetra-hydro-4-oxo-6-benzyl-pyrido[4,3-d]pyrimidin

### Herstellung der Ausgangsmaterialien

a) 3,5,7,8-Tetrahydro-4-oxo-6-benzyl-pyrido[4,3-d]pyrimidin
   Man ließ 4,7 g Natrium in kleinen Portionen in 250 ml Ethanol abreagieren und tropfte dann bei 5-10°C eine Suspension von 14,2 g (0,05 mol) N-Benzyl-4-piperidon-3-carbonsäuremethylester in Ethanol dazu. Nach 30 minütigem Rühren wurden 6 g (0,075 mol) Formamidinhydrochlorid langsam zugegeben und die Reaktionsmischung 10 h unter Rückfluß erhitzt. Das Lösungsmittel wurde im Vakuum entfernt und der Rückstand in 100 ml Wasser mit 2N Salzsäure auf pH = 6,5 - 7 eingestellt, so daß das Produkt ausfiel. Man saugte die Kristalle ab, trocknete im Vakuumtrockenschrank und isolierte so 8 g (66 %). Fp.: 88°C.
   Analog wurden 3,5,7,8-Tetrahydro-4-oxo-6-benzyl-pyrido-[3,4-d]pyrimidin (Fp.: 199 °C) und 3,5,7,8-Tetrahydro-4-oxopyrido[4,3-d]pyrimidin-6-carbonsäure-1,1-dimethylester (Fp.: 160°C) erhalten.
b) 1-(2-Methoxyphenyl)-4-(2-chloreth-1-yl)piperazin
   Man legte eine Lösung von 19,2 g (0,1 mol) o-Methoxyphenylpiperazin und 13,8 g (0.1 mol) Kaliumcarbonat in 200 ml DMF bei Raumtemperatur vor und fügte nach 30 min 30 ml (0,36 mol) 1-Brom-2-chlorethan zu. Es wurde 2 h bei Raumtemperatur gerührt. Nach dem Eingießen in Eiswasser extrahierte man mit Methyltertbutylether, wusch die organischen Phasen mit Wasser, trocknete mit Natriumsulfat und engte anschliessend ein. Aus der Lösung des Rückstandes in Essigester wurde durch Zugabe 30 %iger Isopropanol/HCl Lösung das Hydrochlorid gefällt, welches man nach dem Absaugen bei 40°C im Vakuumtrockenschrank trocknete. Man erhielt 17 g (67 %) Substanz. Fp. : 200°C.
   In analoger Weise wurden 1-(2-Methoxyphenyl)-4-(3-chlorprop-1-yl)piperazin (Fp.: 217°C, Hydrochlörid), 1-(3,4-Methylphenyl)-4-(2-chloreth-1-yl)piperazin (Fp.: 260°C, Hydrochlorid), 1-(2-pyrimidyl)-4-(2-chloreth-1-yl)piperazin (Fp.: 270°C, Hydrochlorid), l-(Napht-l-yl)-4-(3-chlorprop-1-yl)piperazin (Fp.: 217°C, Hydrochlorid), erhalten.

Für die Herstellung der Piperazine sind zwei Beispielsynthesen im folgenden angegeben.

### l-Tetralin-5-yl-piperazin

Man erhitzte 14,7 g (0,1 mol) 5-Aminotetralin mit 18 g (0,11 mol) Bis(β-chlorethyl)aminhydrochlorid in 300 ml n-Butanol 48 h unter Rückfluß, fügte nach dem Abkühlen 5,4 g Nairiumcarbonat hinzu und erhitzte nochmals 20 h unter Rückfluß. Durch Abkühlen entstehender Niederschlag wurde abgesaugt, in Wasser aufgenommen und mit 2N Natriumhydroxidlösung versetzt. Man extrahierte die wässrige Phase mit Essigester, wunsch mit Wasser, trocknete über Natriumsulfat und engte im Vakuum ein. 10,7 g (50 %) des Produktes konnten so als Öl isoliert werden.

### 4-Piperazin-l-yl-isochinolin

Es wurden 4,51 g (21.7 mmol) 4-Bromisochinolin, 4,65 g (25,0 mmol) Piperazin-N-carbonsäure-t-butylester, 0,1 g (0.11 mmol) Tris-(dibenzylidenaceton)-dipalladium, 0,11 g (0,18 mmol) 2,2'-Bis-(diphenylphosphino)-1,1'-binaphthyl und 2,92 g (30,4 mmol) Natrium-t-butylat in 50 ml Toluol zusammengegeben und 2 h bei 75°C gerührt. Man gab die Reaktionsmischung auf Eis/Kochsalz, extrahierte mit Essigester, trocknete die organische Phase über Natriumsulfat und entfernte das Lösungsmittel am Rotationsverdampfer. Das Produkt kristallisierte aus, wurde abgesaugt und mit Pentan gewaschen. Man erhielt 5,5 g (81 %) des Bocgeschützten Piperazins (Fp.: 111°C). Es wurden 5,2 g (16,6 mmol) dieser Substanz in 17 ml Dichlormethan aufgenommen und bei 0°C langsam mit 17 ml (0,22 mol) Trifluoressigsäure versetzt.. Man ließ 4 h bei 0°C rühren, goß auf Eiswasser und extrahierte mit Dichlormethan. Die wässrige Phase wurde filtriert, alkalisch eingestellt und mit Dichlormethan extrahiert. Nach dem Trocknen über Natriumsulfat und dem weitgehenden Entfernen des Lösungsmittels verdünnte man mit Diethylether und fällte das Hydrochlorid mit etherischer Salzsäure. Man erhielt 3,2 g (67 %) des Produktes. (Fp.: 293°C).

In Analogie zu den beiden beschriebenen Verfahren wurden die folgenden Verbindungen hergestellt: 1-Naphth-1-yl-azepan (85°C, Hydrochlorid), 1-Naphth-1-ylmethyl-piperazin (Öl), 4-Piperazin-1-yl-indan (Öl), 1-Naphth-1-yl-piperazin (82°C), 4-Chlor-1-piperazin-1-yl-phthalazin (205 °C, Zers.) und 4-Piperazin-1-yl-chinazolin (320°C, Hydrochlorid). Weitere Derivate waren kommerziell erhältlich.

### Herstellung des Endproduktes

Zu einer Lösung von 2,4 g (10 mmol) Tetrahydropyridopyrimidin [a)] in 40 ml DMF wurden 2,9 g (10 mmol) Chlorethylpiperazin [b)] und 2,8 g (20 mmol) Kaliumcarbonat gegeben. Nach zweistündiger Reaktion bei 90°C goß man die Reaktionsmischung auf Eiswasser und extrahierte mit Essigester. Die organische Phase wurde mit gesättigter Kochsalzlösung gewaschen, über Natriumsulfat getrocknet und das Lösungsmittel im Vakuum entfernt. Das zurückbleibende Öl nahm man in Aceton auf und fällte das Hydrochlorid mit Isopropanol/HCl. Man erhielt 4 g (75 %) des Produktes (Fp.: 205°C).

NMR: CDCl₃ δ 8.0 (s, 1H), 7.4 - 7.2 (m, 5H), 7.1 - 6.8 (m, 4H), 4.0 (t, 2H), 3.8 (s, 3H), 3.7 (s, 2H), 3.5 (s, 2H), 3.1 (br. s, 4H), 2.8 - 2.6 (m, 10H) ppm.

Es wurden in analoger Weise die folgenden Verbindungen erhalten:

### Beispiel 2:

3-[2-[4-(2-methoxyphenyl)-1-piperazinyl]ethyl]-3,5,7,8-tetra-hydro-4-oxo-6-benzyl-pyrido[3,4-d]pyrimidin (Fp.: 181°C, Hydrochlorid).

### Beispiel 3:

3-[3-[4-(2-methoxyphenyl)-1-piperazinyl]propyl]-3,5,7,8-tetra-hydro-4-oxo-6-benzyl-pyrido[4,3-d]pyrimidin (Fp.: 198°C, Hydrochlorid).

### Beispiel 4:

3-[3-[4-(2-methoxyphenyl)-1-piperazinyl]propyl]-3,5,7,8-tetra-hydro-4-oxo-6-benzyl-pyrido[3,4-d]pyrimidin (Fp.: 190°C, Hydrochlorid).

### Beispiel 5:

3-[3-[4-(2-methoxyphenyl)-1-piperazinyl]2-hydroxypropyl]-3,5,7,8-tetrahydro-4-oxo-6-benzyl-pyrido[4,3-d]pyrimidin.

### Beispiel 6:

3-[2-[4-(naphth-1-yl)-1-piperazinyl]ethyl]- 3,5,7,8-tetra-hydro-4-oxo-pyrido[4,3-d]pyrimidin-6-carbonsäure-1,1-dimethylester (Fp.: 170°C, Hydrochlorid).

### Beispiel 7:

3-[2-[4-(isochinolin-4-yl)-1-piperazinyl]ethyl]-3,5,7,8-tetra-hydro-4-oxo-6-benzyl-pyrido[4,3-d]pyrimidin (Fp.: 268°C, Hydrochlorid).

### Beispiel 8:

3-[2-[4-(naphth-1-yl)-1-piperazinyl]ethyl]-3,5,6,7,8-pentahydro-4-oxo-pyrido[4,3-d]pyrimidin (Fp.: 272°C, Hydrochlorid).

### Beispiel 9:

3-[2-[4-(chinazolin-4-yl)-1-piperazinyl]ethyl)-3,5,7,8-tetra-hydro-4-oxo-6-benzyl-pyrido[4,3-d]pyrimidin (Fp.: 258°C, Hydrochlorid).

### Beispiel 10:

3- (2-[4-(naphth-1-yl)-1-piperazinyl]ethyl]-3,5,7,8-tetra-hydro-4-oxo-6-benzyl-pyrido[4,3-d]pyrimidin (Fp.: 227°C, Hydrochlorid).

### Beispiel 11:

3-[2-[4-(naphth-1-yl)-tetrahydro-1,2,3,6-pyridin-1-yl]eth-1-yl]-3,5,7,8-tetrahydro-4-oxo-6-benzyl-pyrido[4,3-d]pyrimidin (Fp.: 216°C, Hydrochlorid).

### Synthese der Ausgangsmaterialien

a) N-Boc-4-(Trifluormethansulfonyloxy)-tetrahydro-1,2,3,6-pyridin
   Eine Lösung von 13,2 g (0,13 mol) Diisopropylamin in 200 ml THF wurde bei -78°C mit 100 ml nBuLi (1.6M in Hexan) deprotoniert und nach 30 Minuten bei dieser Temperatur 20,0 g (0,1 mol) des in 50 ml THF gelösten N-Boc-piperidon zugetropft. Nach weiteren drei Stunden bei -78°C gab man eine Lösung von 39,3 g (0,11 mol) N,N,-Bistrifluormethansulfonylanilin in 50 ml THF zu und ließ über Nacht auf Raumtemperatur kommen. Zur Aufarbeitung versetzte man mit Wasser, extrahierte mit Ether, wusch die organischen Phasen mit NaHCO₃-Lösung und Wasser, trocknete über Natriumsulfat und engte das Lösungsmittel ein. Das Rohprodukt wurde mittels Flashchromatographie (Kieselgel, Laufmittel Heptan/Essigester = 3/1) gereinigt.
   Ausbeute: 20,2 g (60 % d. Th.)
   1H-NMR:(270 MHz, CDCl₃)δ= 1.4 (s, 9H); 2.4(m, 2H); 3.6 (t, 2H) ; 4.1 (m, 2H); 5.8 (m, 1H)ppm
b) N-Boc-4-Naphth-1-yl-tetrahydro-1,2,3,6-pyridin
   14,7g (44.4 mmol) der vorstehend beschriebenen Verbindung gelöst in 115 ml Dimethoxyethan wurden nacheinander 22 ml 2M Natriumcarbonat-Lösung, 7,63 g(44.4 mmol) Naphthyl-1-boronsäure, 4,13 g (97,6 mmol) Lithiumchlorid, 0,85 g (4,44 mmol) Kupfer(I)jodid und 2,1 g (1,77 mmol) Tetrakistriphenylpalladium zugesetzt und 4h zum Sieden erhitzt. Zur Aufarbeitung wurde unter Zusatz von wässriger Ammoniak-Lösung mit Wasser und Essigsäureethylester extraktiv aufgearbeitet, über Natriumsulfat getrocknet und den nach Evaporation des Lösungsmittels erhaltene Rückstand mittels Flashchromatographie (Kieselgel, Laufmittel Heptan/Essigester = 4/1) aufgereinigt.
   Ausbeute: 8,2 g (57 % d. Th.)
   1H-NMR (270 MHz, CDCl₃): δ= 1.4 (s, 9H); 2.5 (m, 2H); 3.7(t, 2H); 4.1 (m, 2H); 5.8 (m, 1H); 7.2-7.5(m, 3H); 7.3-8.0 (m, 3H) ppm.
c) 4-Naphth-1-yl-tetrahydro-1,2,3,6-pyridin
   7,84 g (25,3 mmol) N-Boc-4-Naphth-1-yl-tetrahydro-1,2,3,6-pyridin wurden über Nacht mit 200 ml etherischer Salzsäure bei Raumtemperatur gerührt, das ausgefallene Produkt abfiltriert und getrocknet.
   Ausbeute: 5,5 g (88 % d. Th).
d) Darstellung der Endverbindung
   0,51 g (2 mmol) 4-Naphth-l-yl-tetrahydro-1,2,3,6-pyridin gelöst in 30 ml trockenem DMF wurden mit 0,61 g (2 mmol) 3-(2-Chlor-eth-1-yl)-3,5,7,8-tetrahydro-4-oxo-6-benzyl-pyrido[4,3-d]pyrimidin und mit 2 ml (17 mmol) Triethylamin versetzt und 5 h bei 120°C gerührt. Die mit Ether verdünnte organische Phase wurde mit Wasser gewaschen, über Natriumsulfat getrocknet und das Lösemittel im Vakuum entfernt. Man reinigte das erhaltene Rohprodukt chromatographisch und erhielt durch Fällung des Salzes mit etherischer Salzsäurelösung einen weißen Feststoff.
   Ausbeute: 0,2 g (20 % d. Th.)
   Fp.: 237°C.

### Beispiel 12

3-[2-[4-(naphth-1-yl)-piperidin-1-yl]eth-1-yl]-3,5,7,8-tetra-hydro-4-oxo-6-benzyl-pyrido[4,3-d]pyrimidin

4-Naphth-l-yl-piperidin

3,7 g (15,3 mmol) 4-Naphth-1-yl-tetrahydro-1,2,3,6-pyridin gelöst in Methanol wurden unter Zugabe von 0.8 g Palladium auf Kohlenstoff mit Wasserstoff bei Raumtemperatur über 48h hydriert. Man filtrierte vom Katalysator ab, engte das Lösungsmittel ein.

Ausbeute: 1,8 g (56 % d. Th.)

1H-NMR (270 MHz, CDCl₃) δ = 1.6-1.8 (m, 2H); 2.0 (m, 2H); 2.9 (dt, 2H); 3.3 (d, 2H); 3.5 (tt, 1H); 7.4-7.6 (m, 4H); 7.7 (d, 1H); 7.9 (d, 1H); 8.1 (d, 1H) ppm.

### Darstellung der Endverbindung

0,42 g (2 mmol) 4-Naphth-1-yl-piperidin geöst in 30 ml trockenem DMF wurden mit 0,61 g (2 mmol) 3-(2-Chlor-eth-1-yl)-3,5,7,8-tetrahydro-4-oxo-6-benzyl-pyrido[4,3-d]pyrimidin und mit 2 ml (17 mmol) Triethylamin versetzt und 5 h bei 120°C gerührt. Die mit Ether verdünnnte organische Phase wurde mit Wasser gewaschen, über Natriumulfat getrocknet und das Lösemittel im Vakuum entfernt. Man reinigte das erhaltene Rohprodukt chromatographisch und erhielt durch Fällung des Salzes mit etherischer Salzsäurelösung einen weißen Feststoff.

Ausbeute: 0,24 g (27 % d. Th.)

1H-NMR (270 MHz, CDCl₃)δ = 8.3 (s, 1H), 8.0 (d,1H), 7.8 (d, 1H), 7.7 (t, 1H), 7.5 - 7.2 (m, 9H), 4.5 (s, 2H), 4.0 (s, 2H), 3.7 - 2.3 (m, 15H), 2.1 (d, 2H) ppm.

In der folgenden Tabelle sind weitere bevorzugte erfindungsgemäße Verbindungen der Formel I aufgeführt.

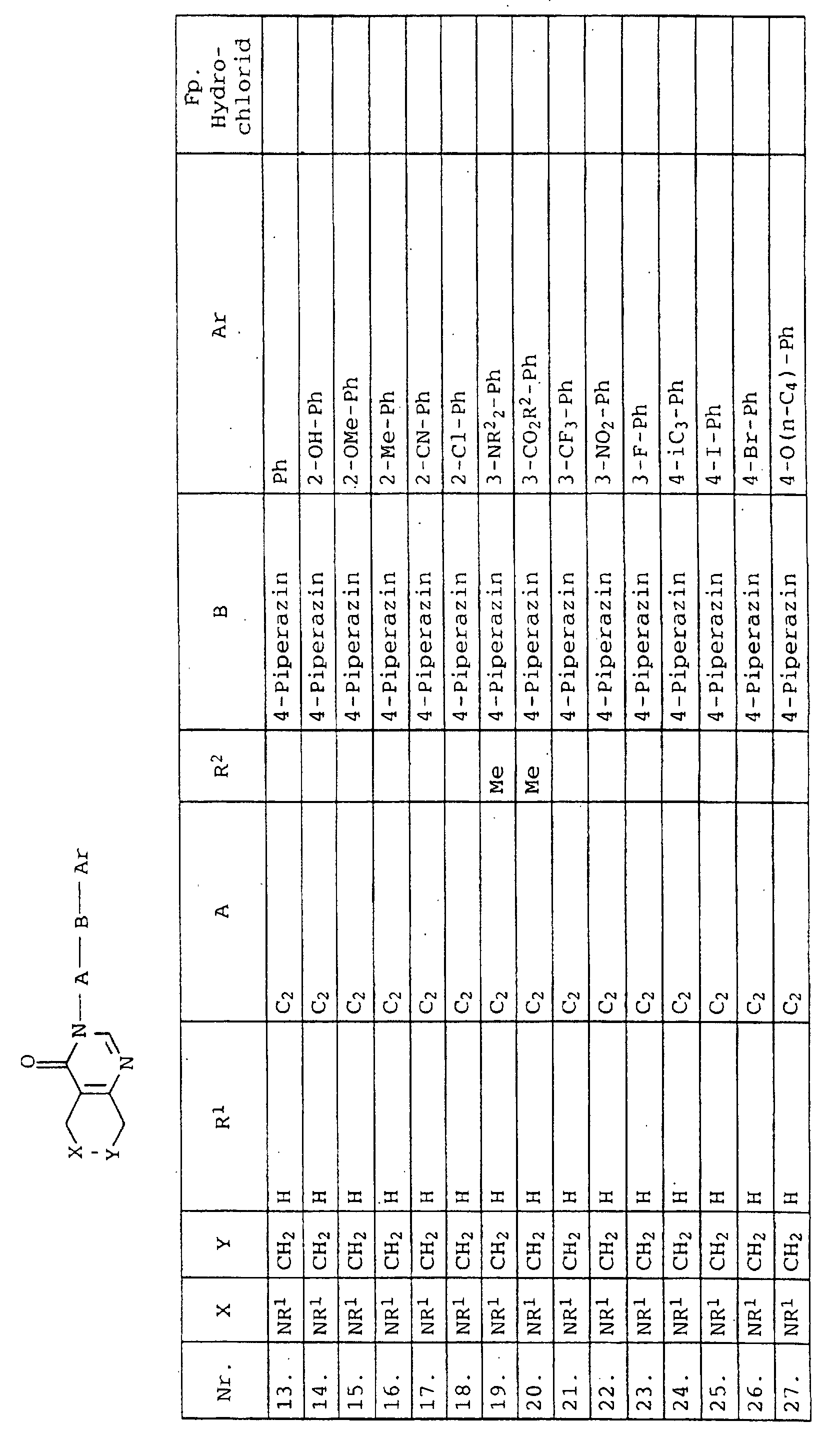

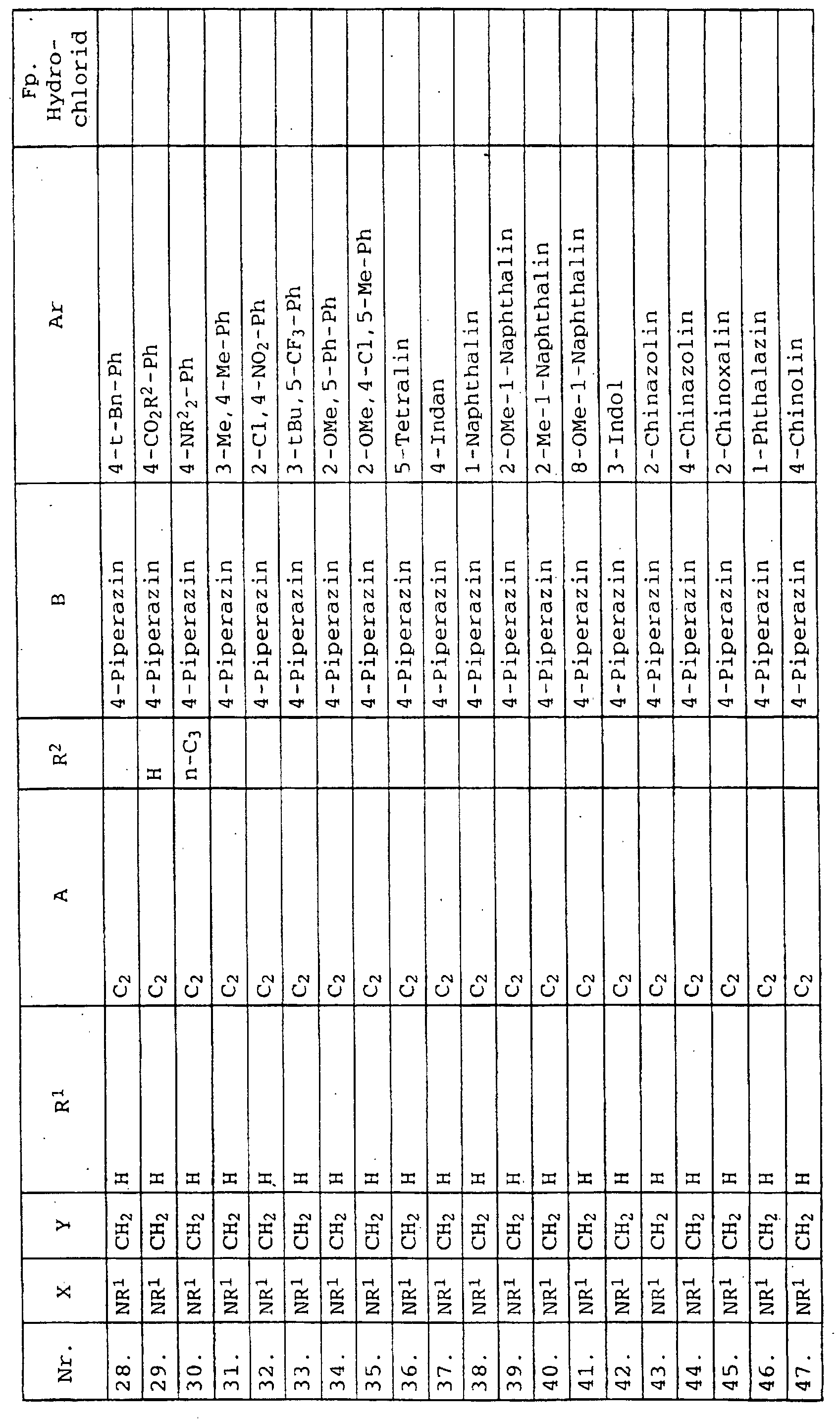

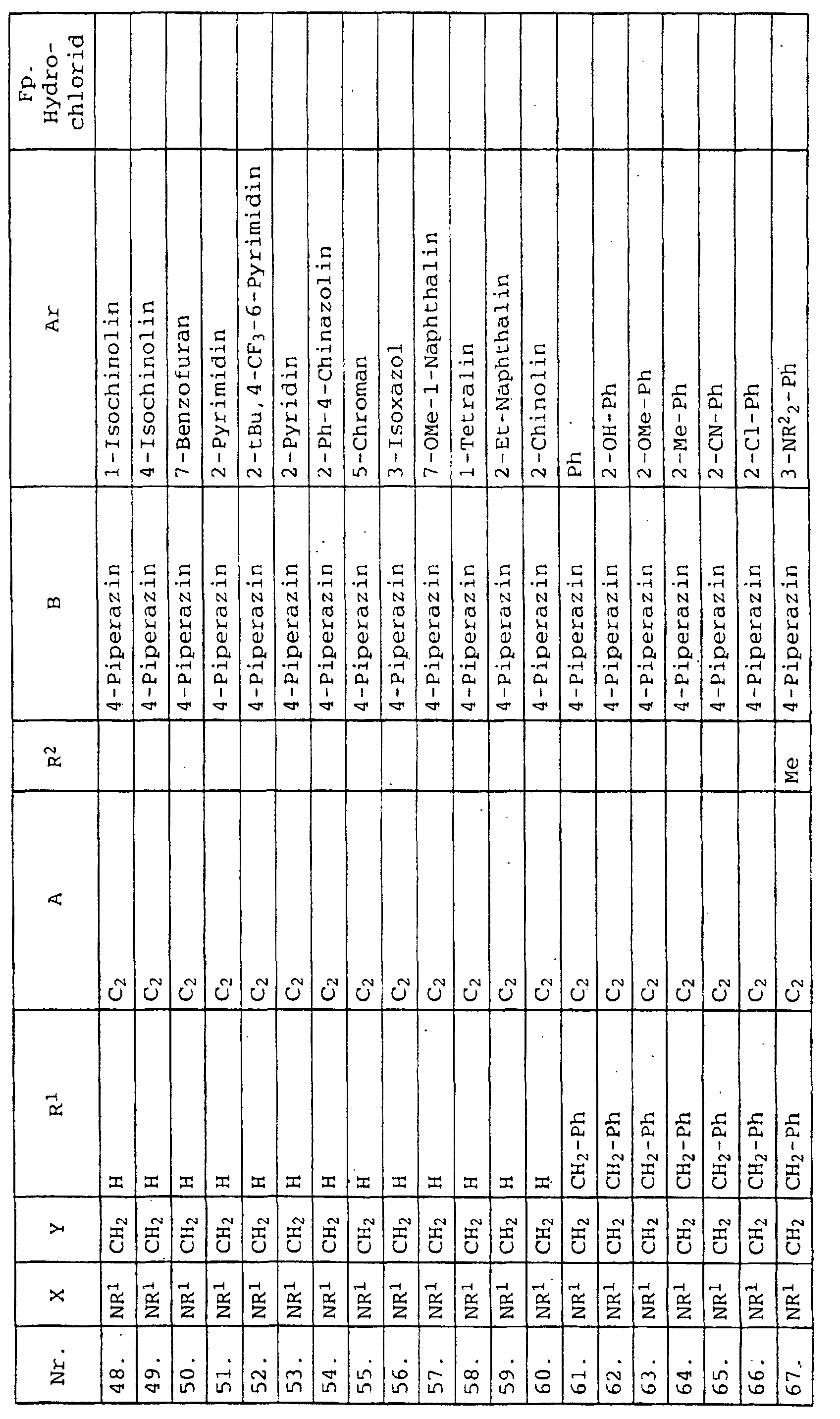

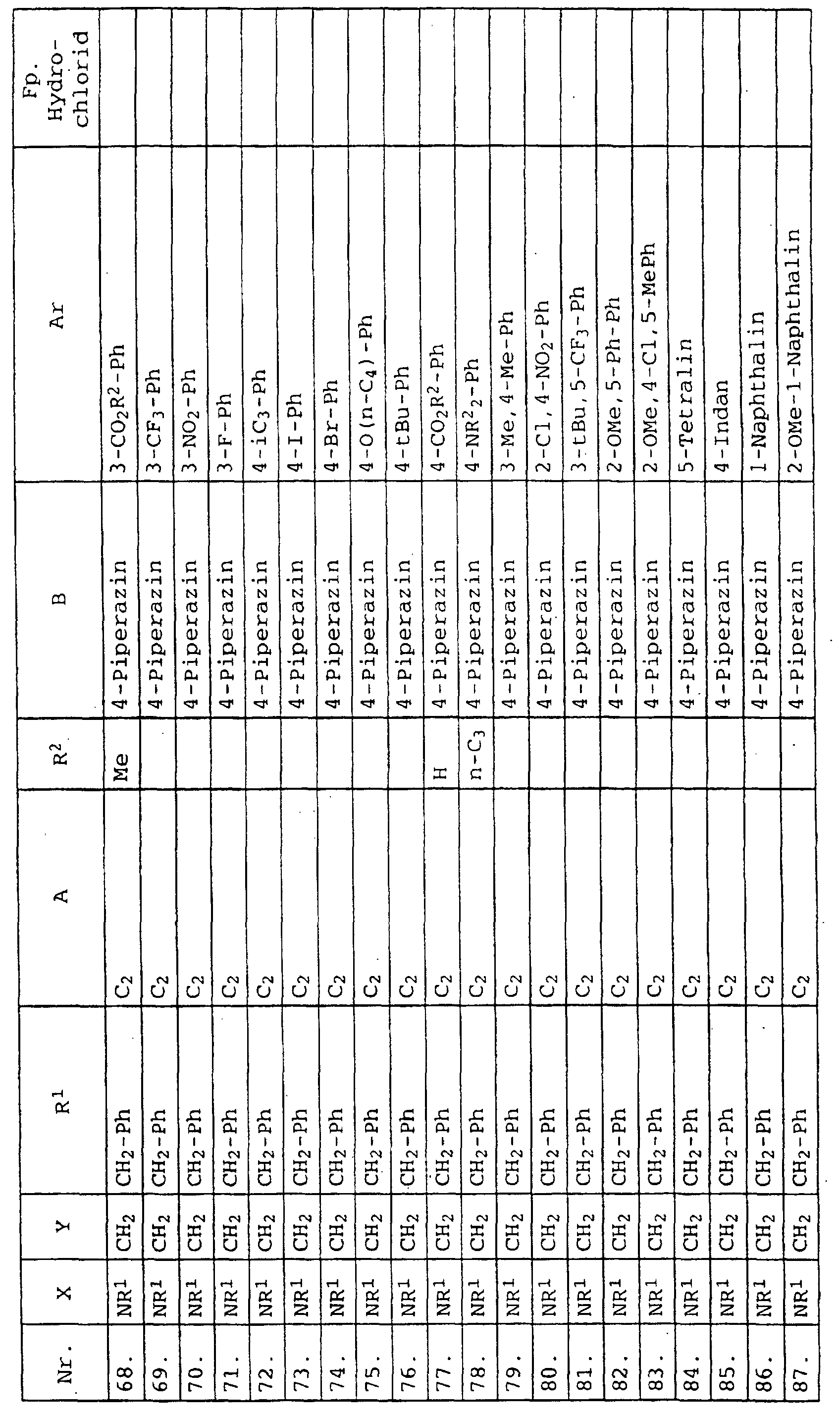

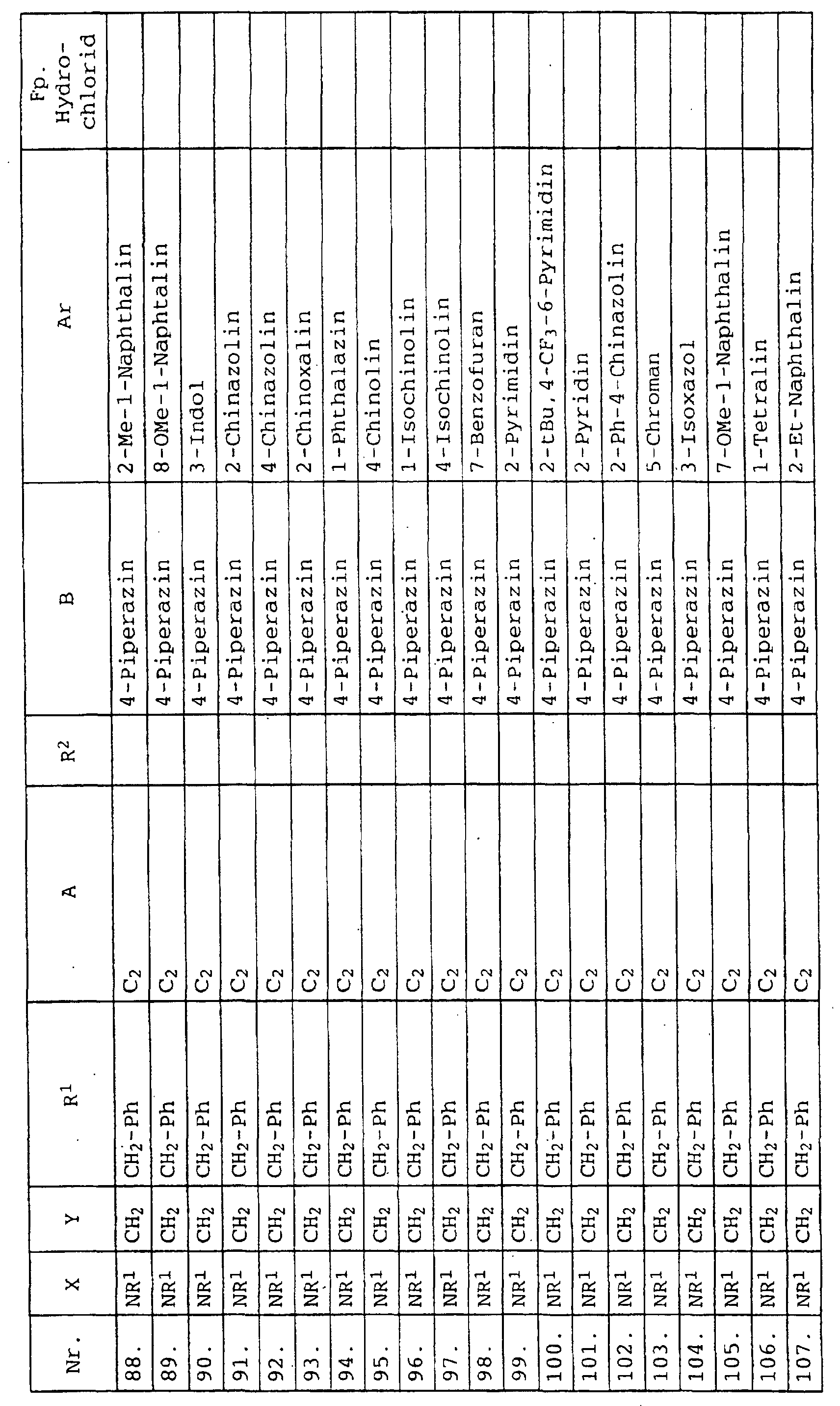

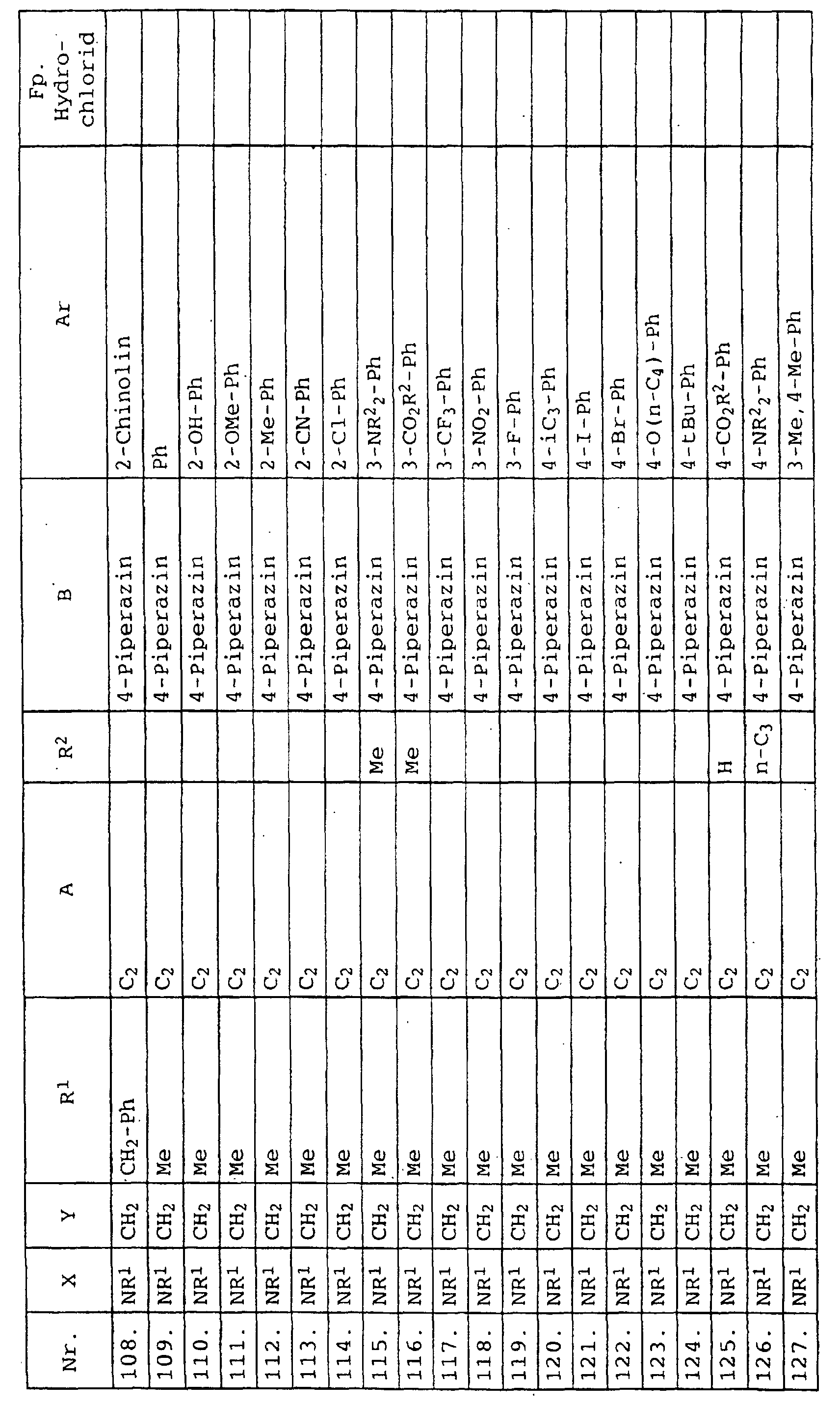

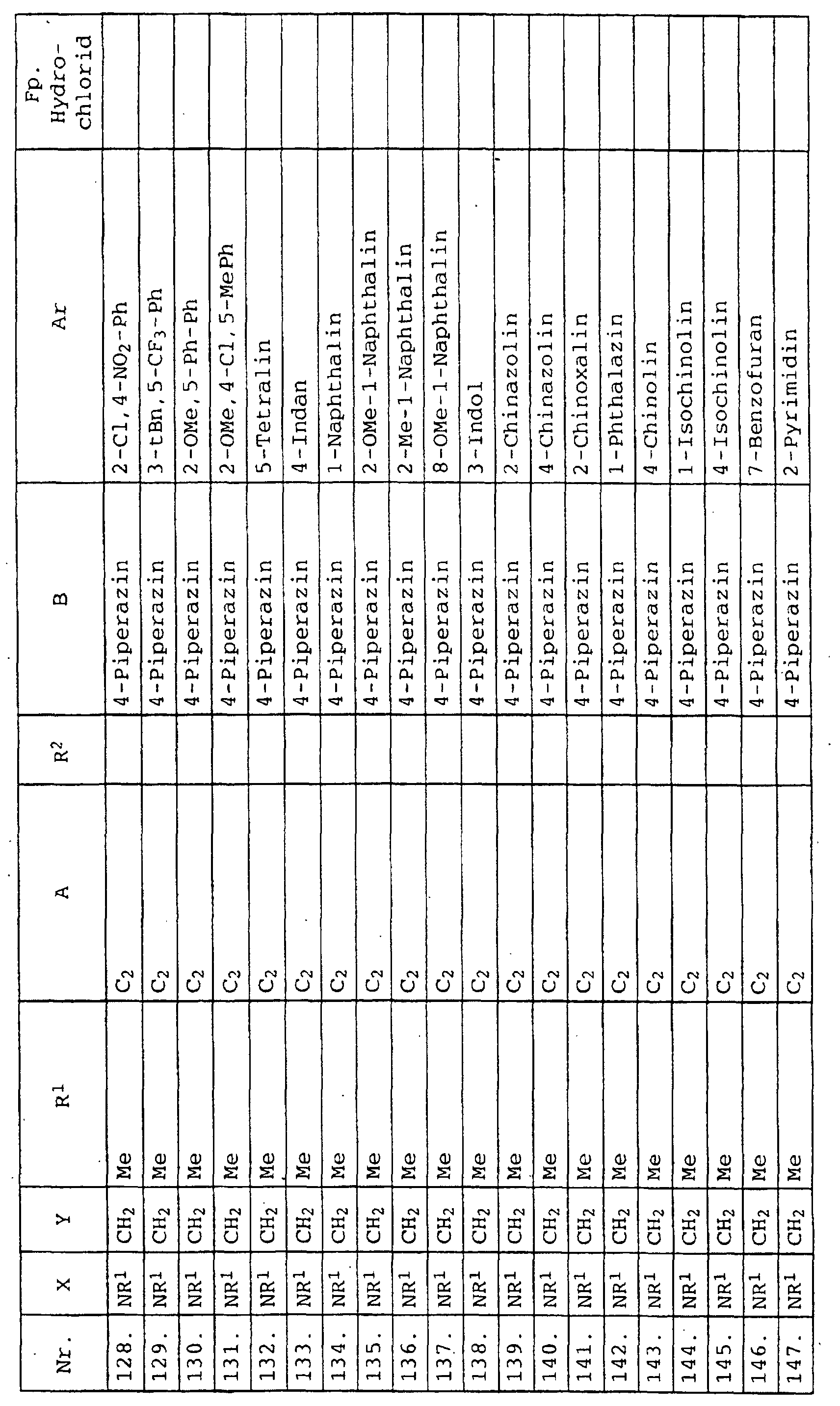

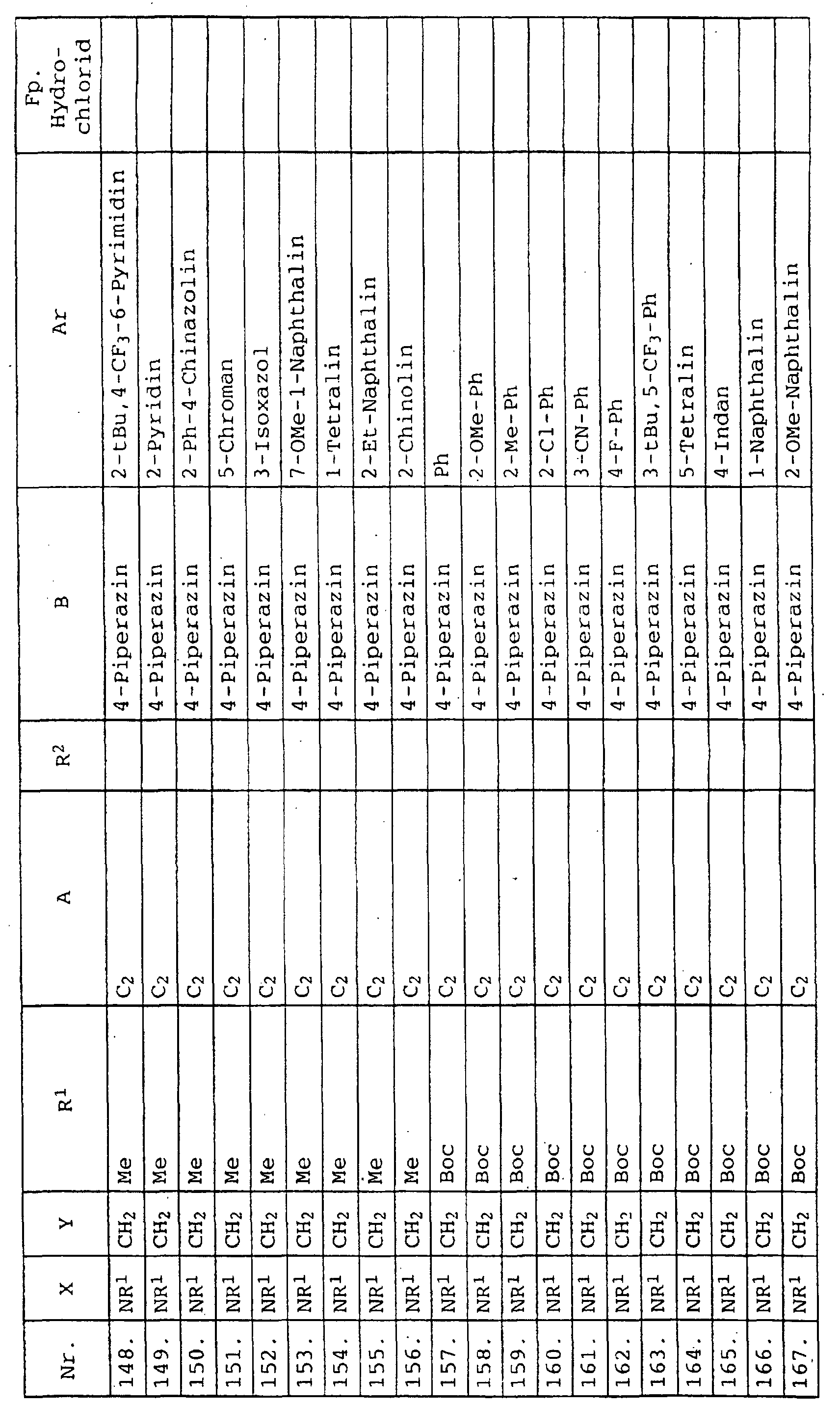

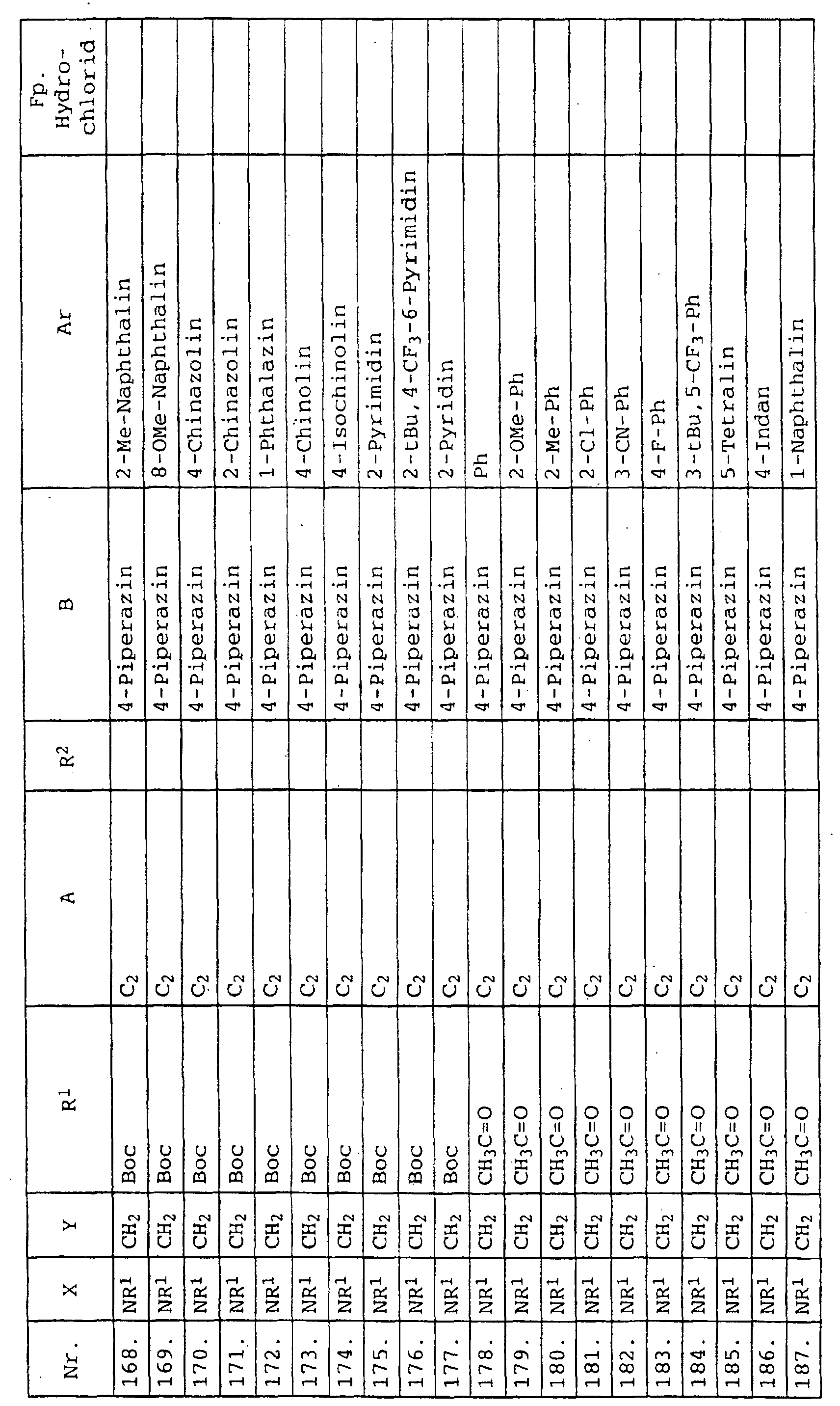

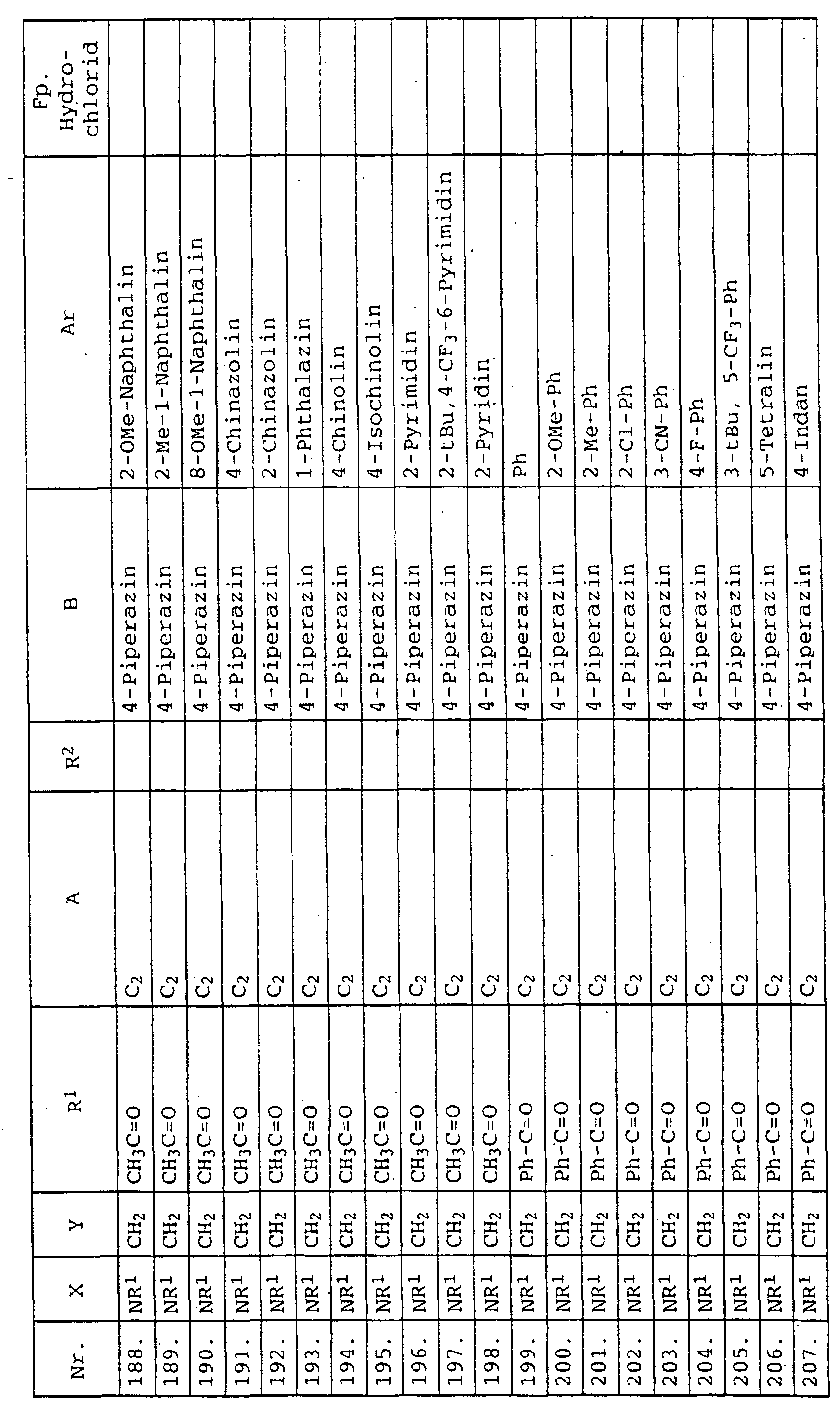

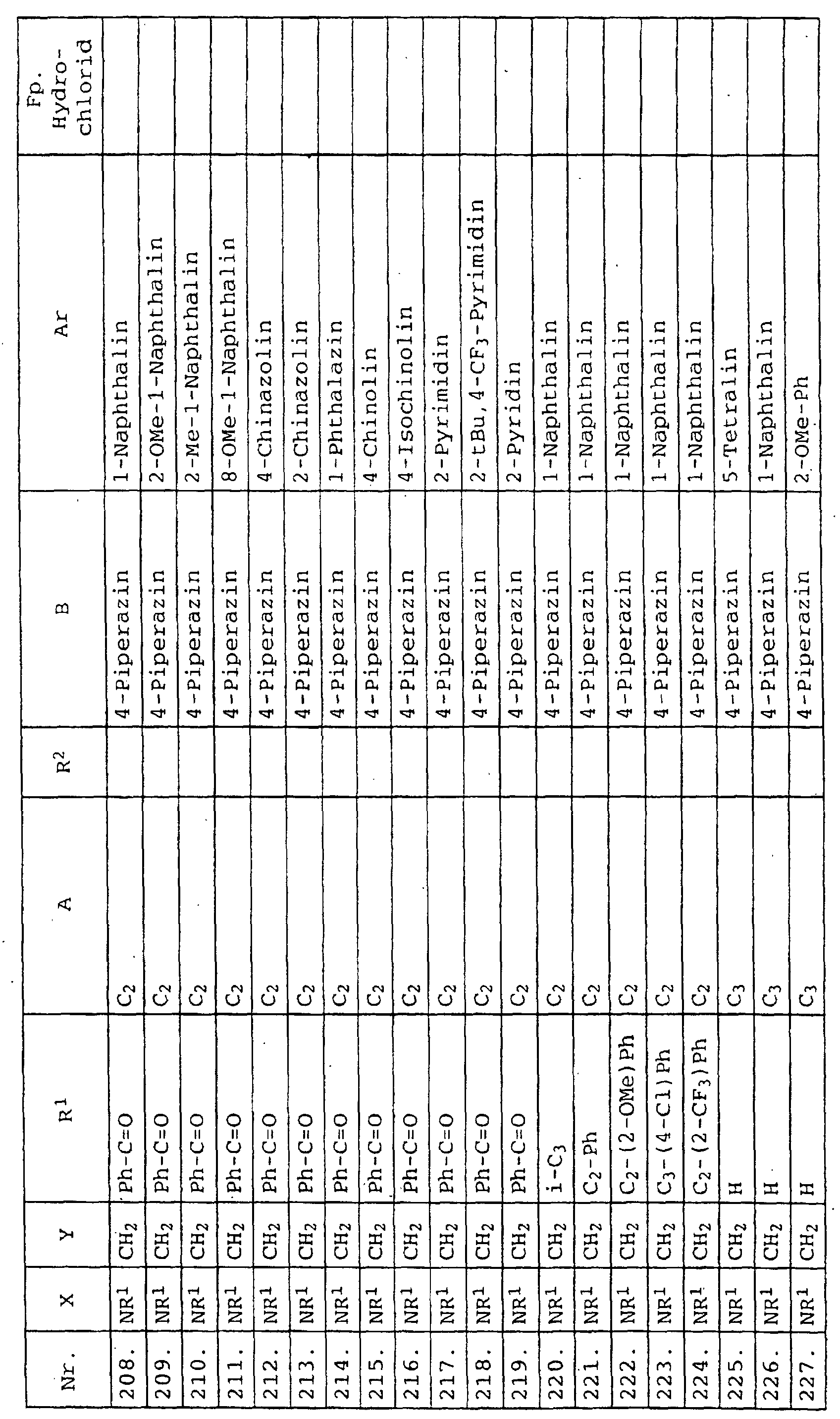

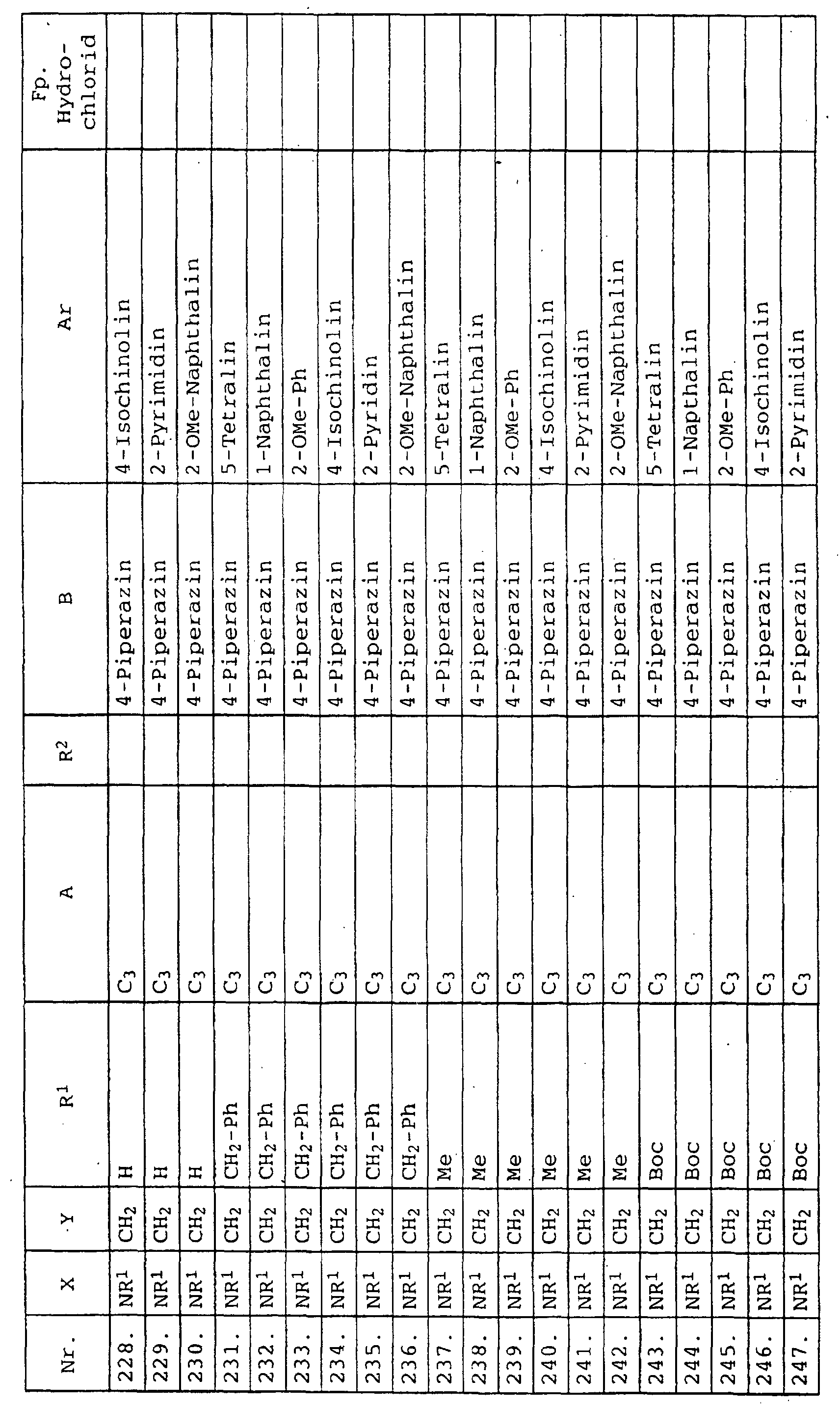

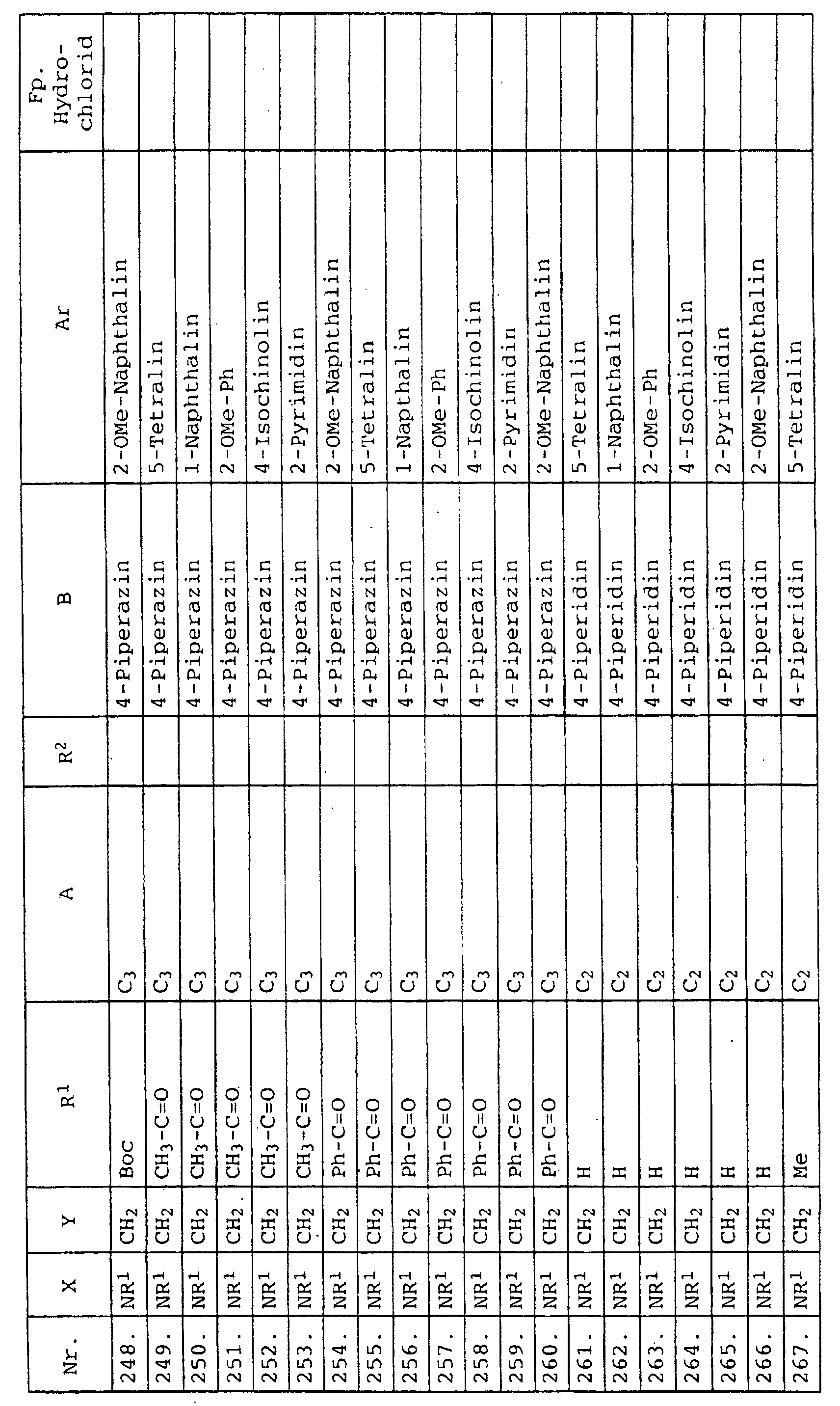

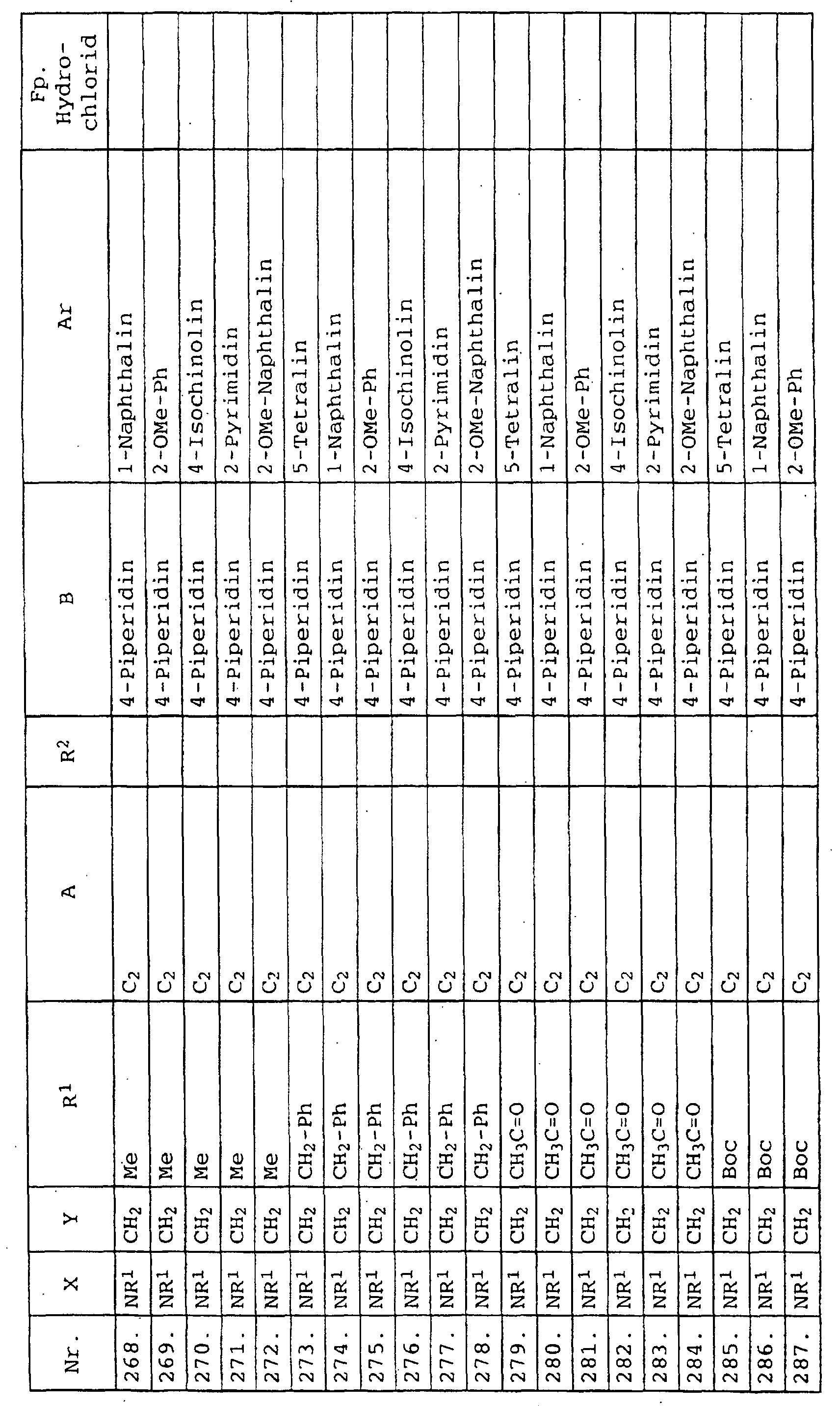

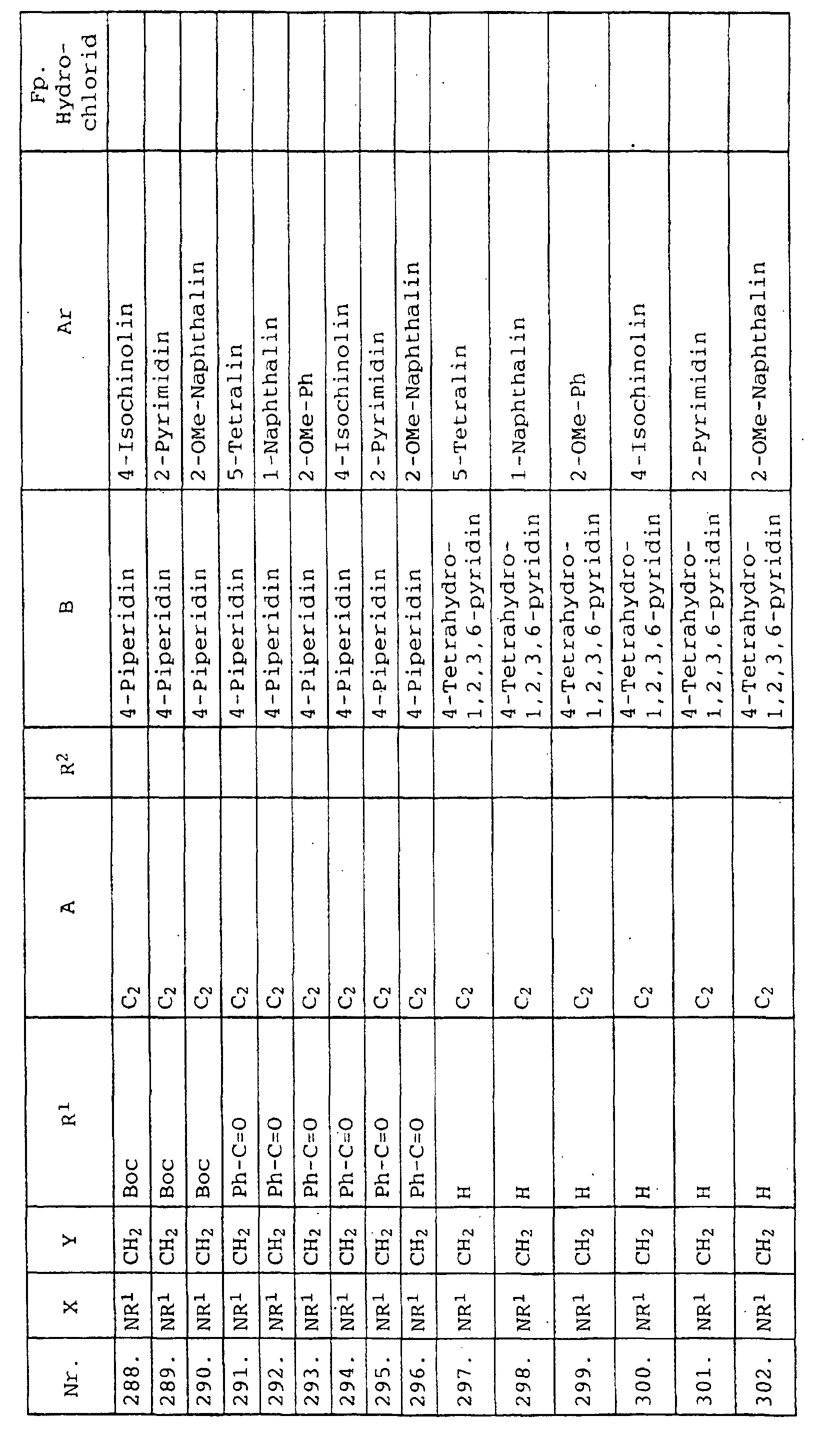

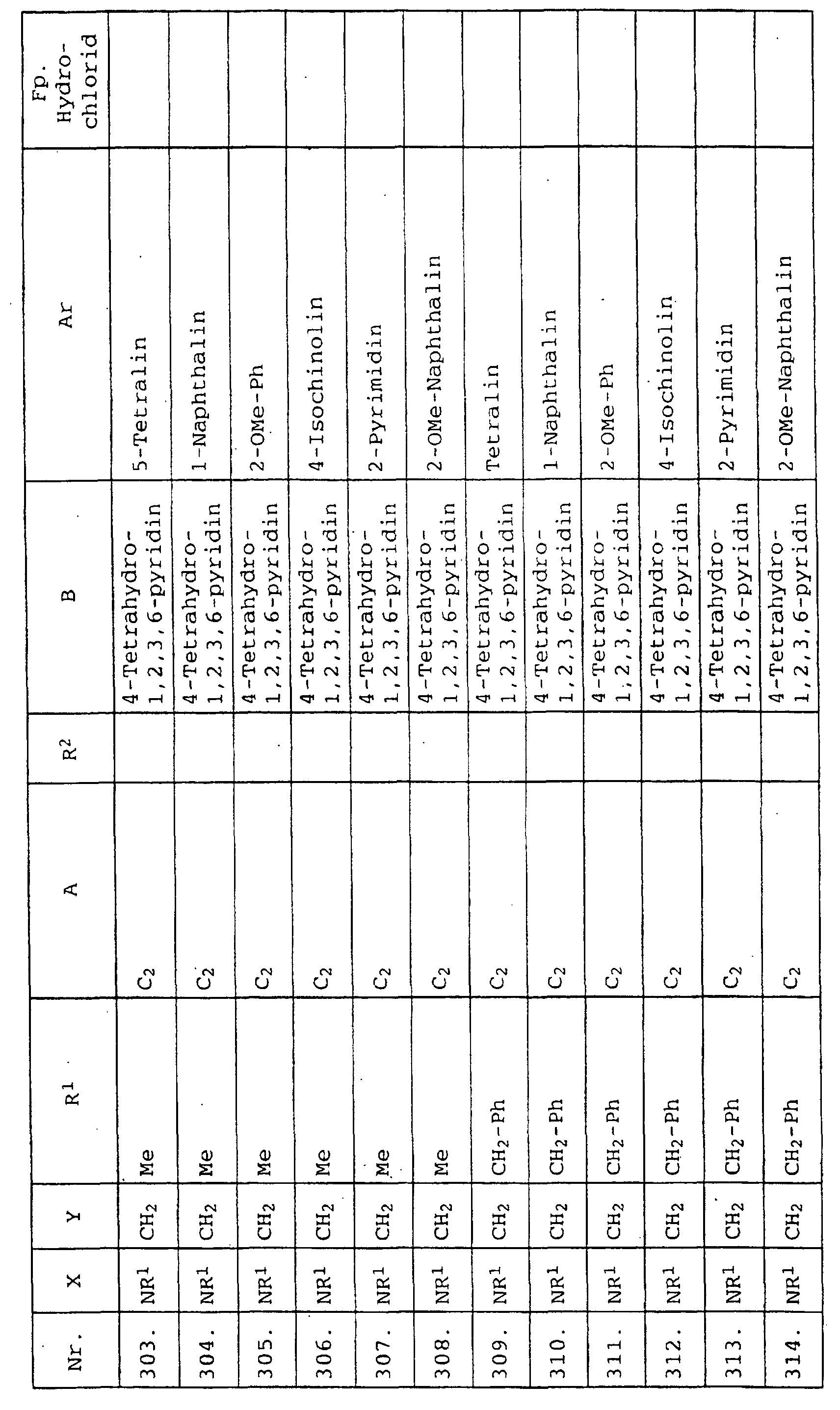

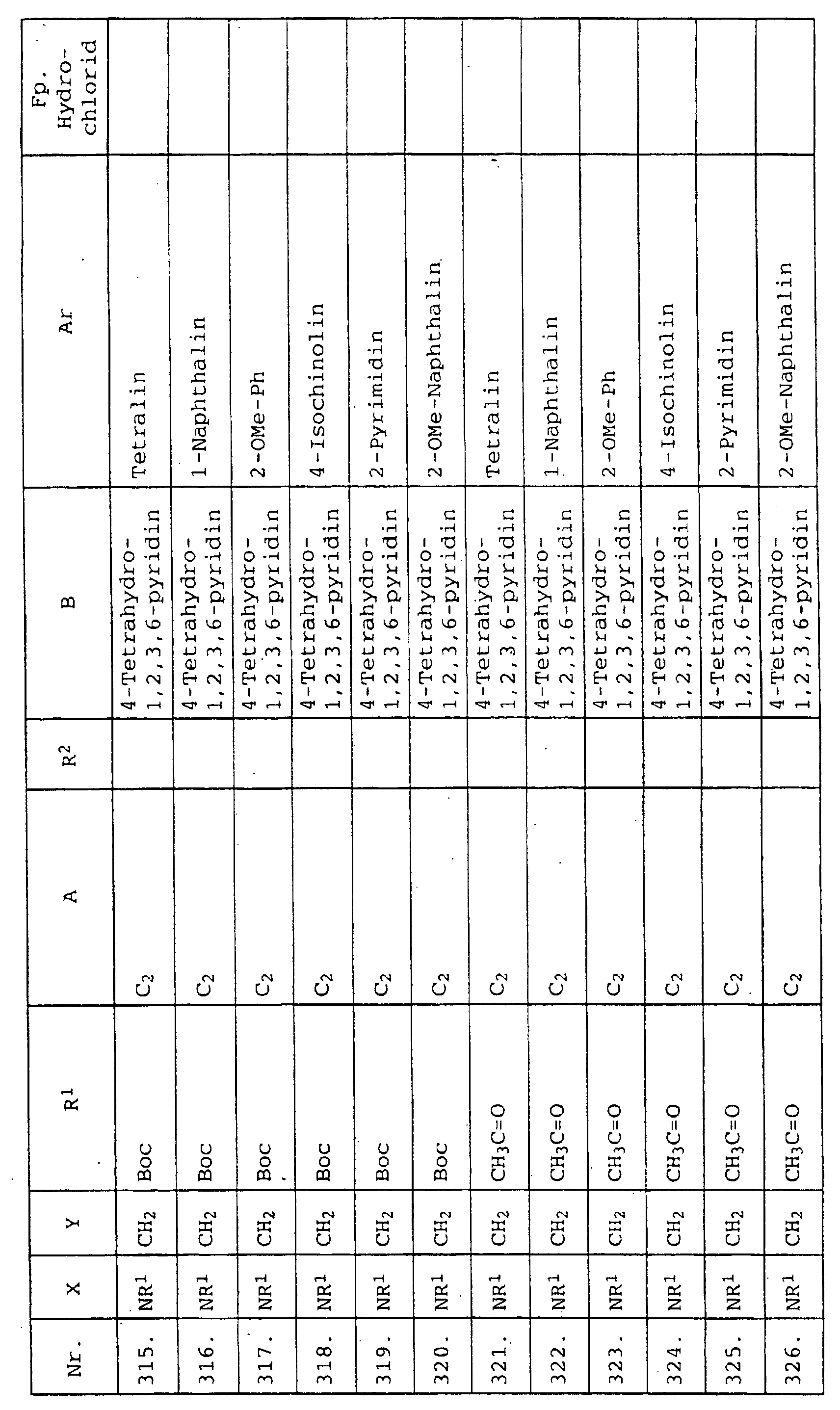

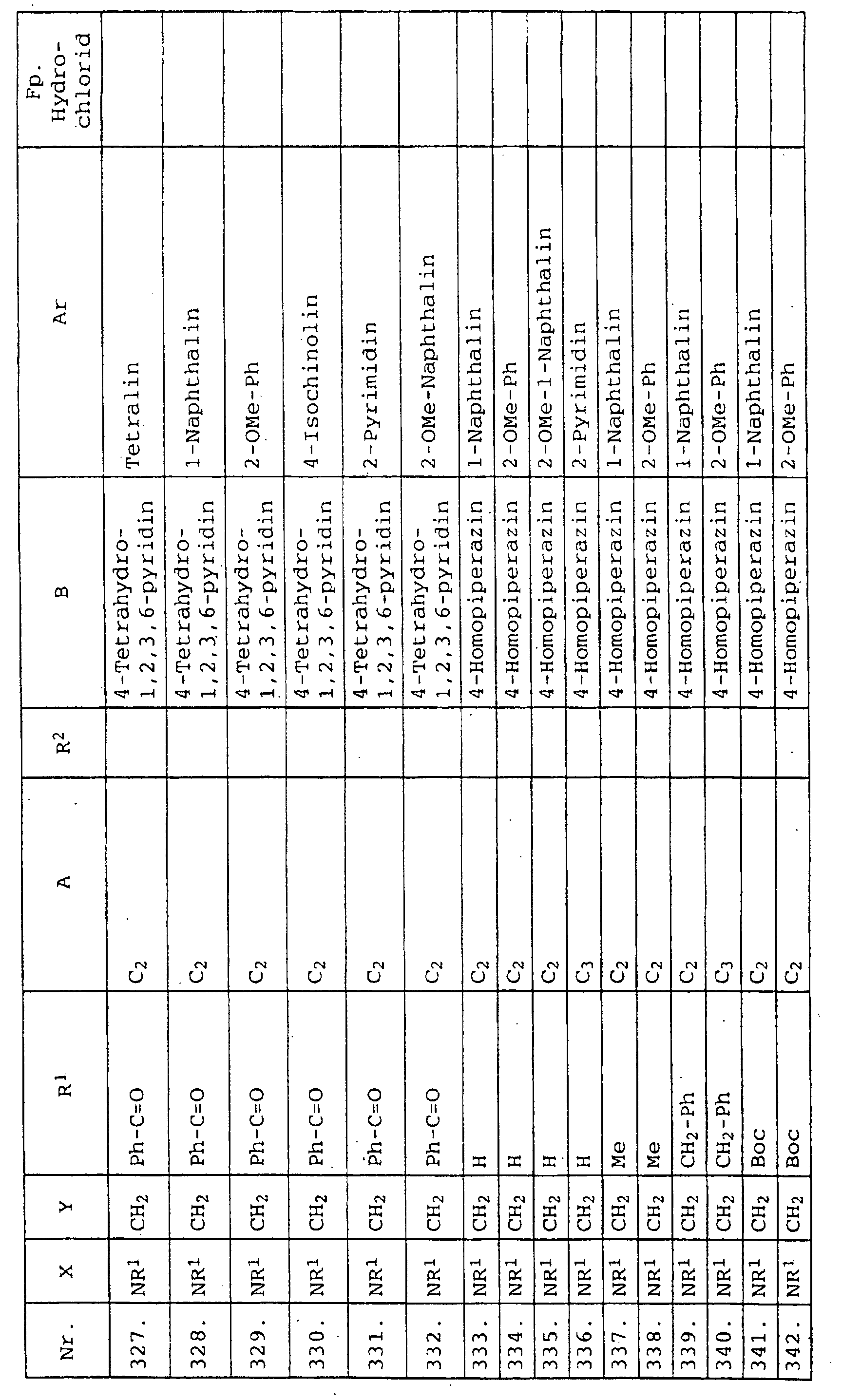

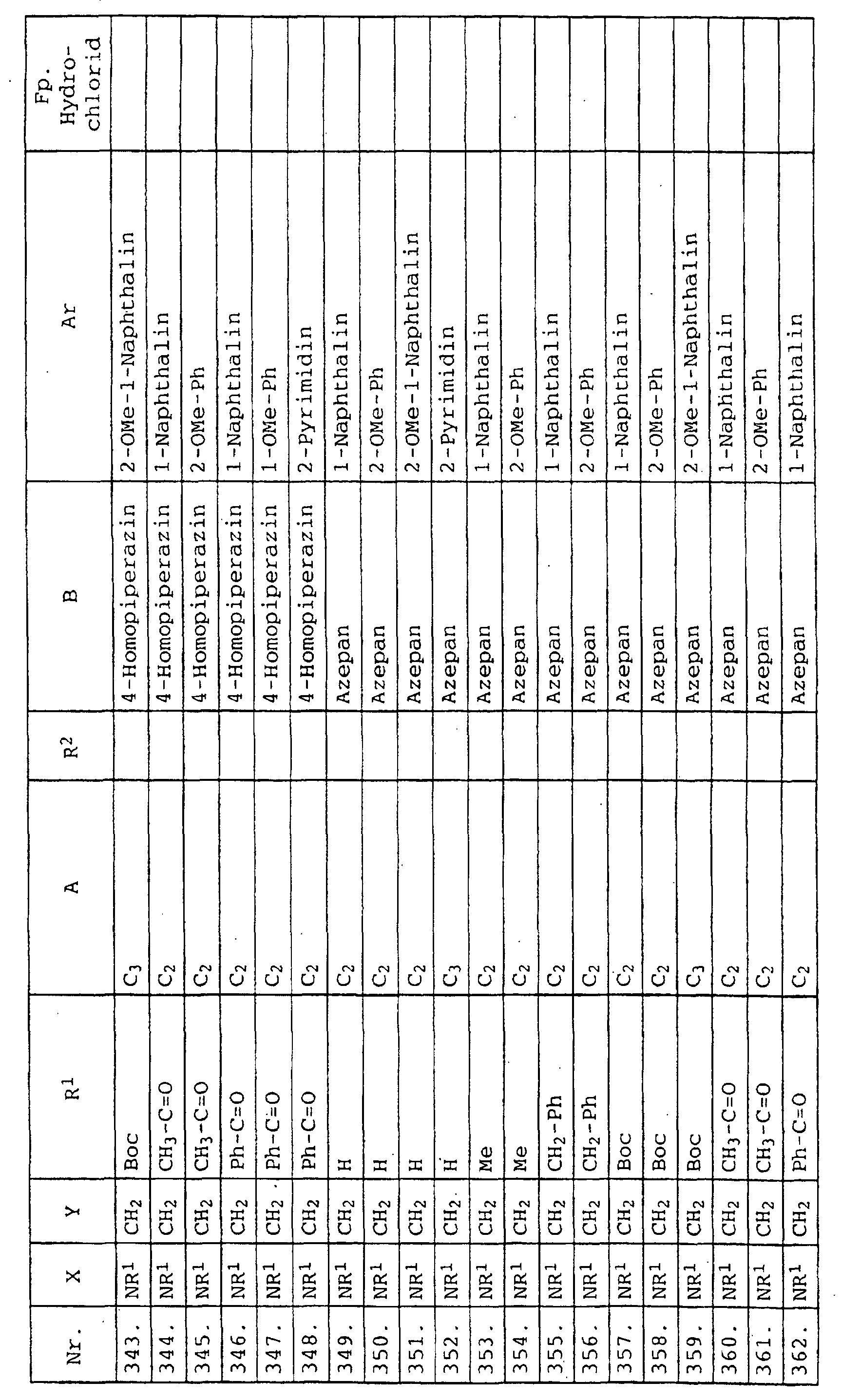

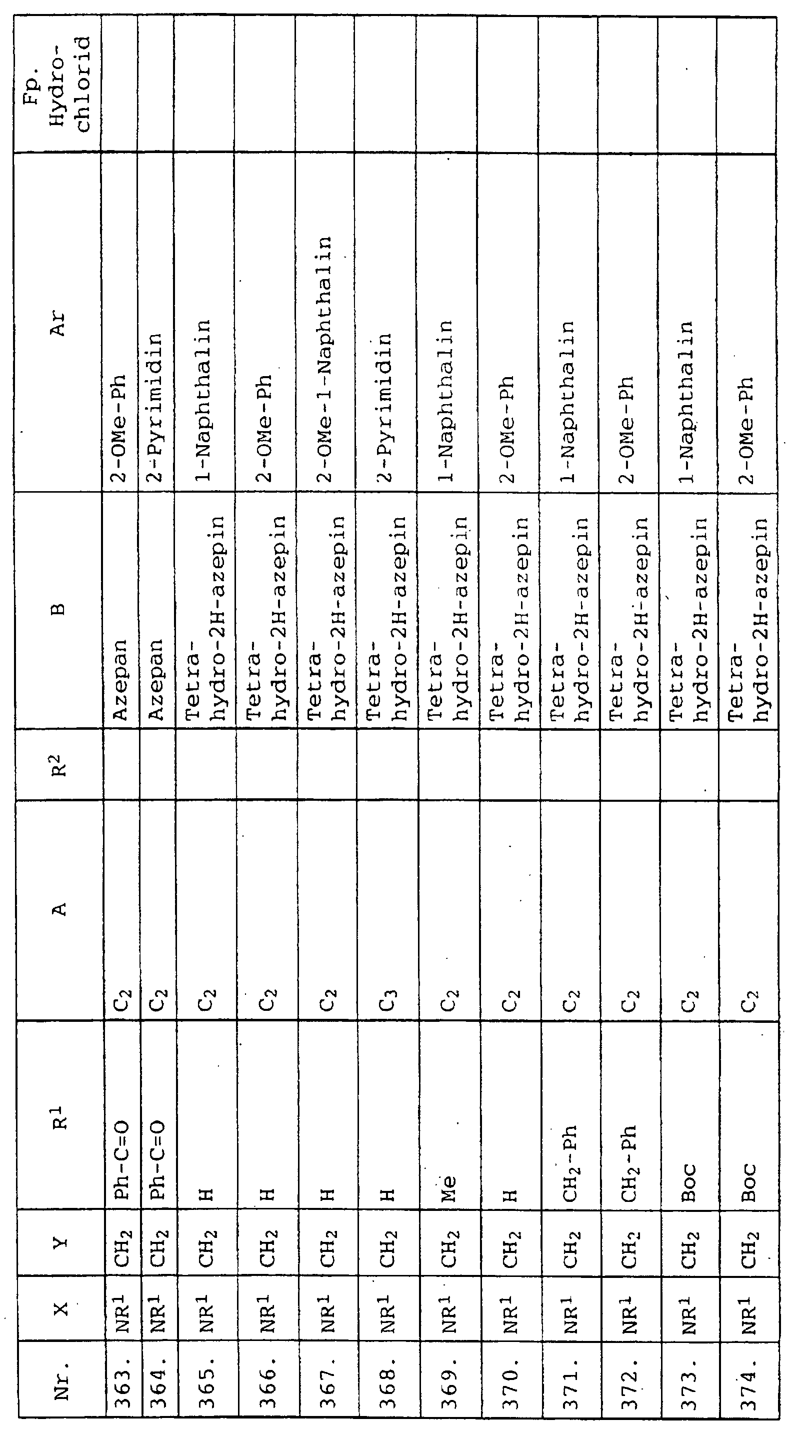

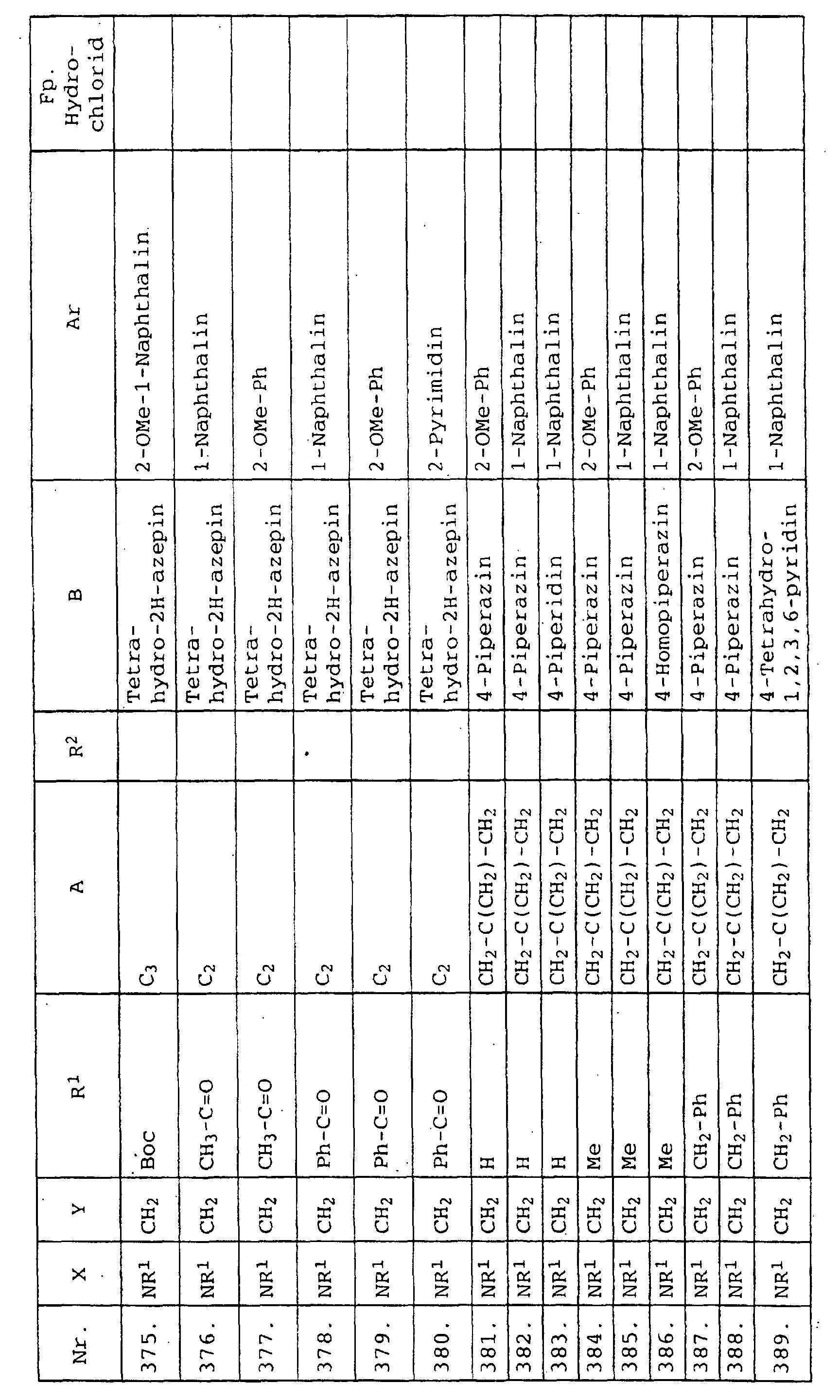

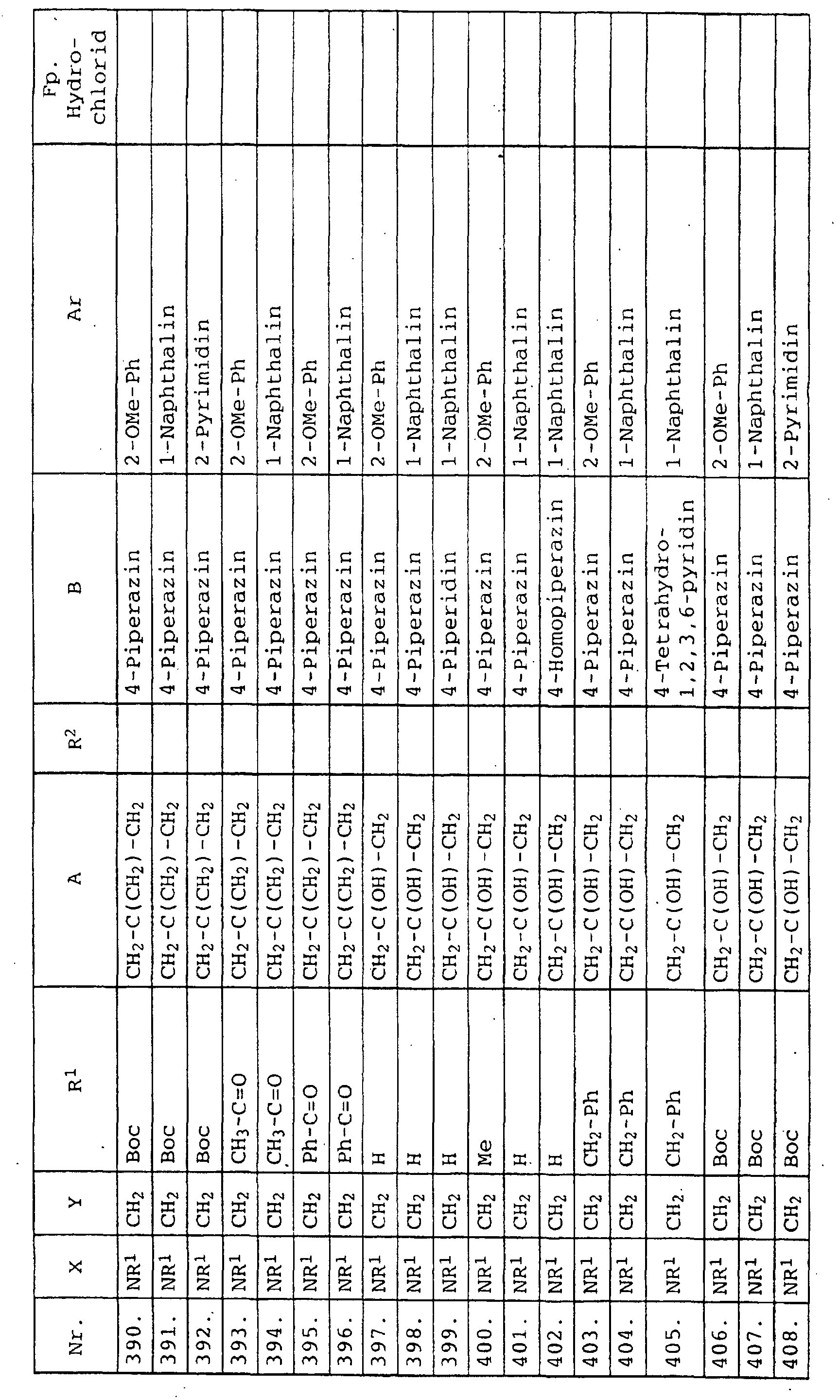

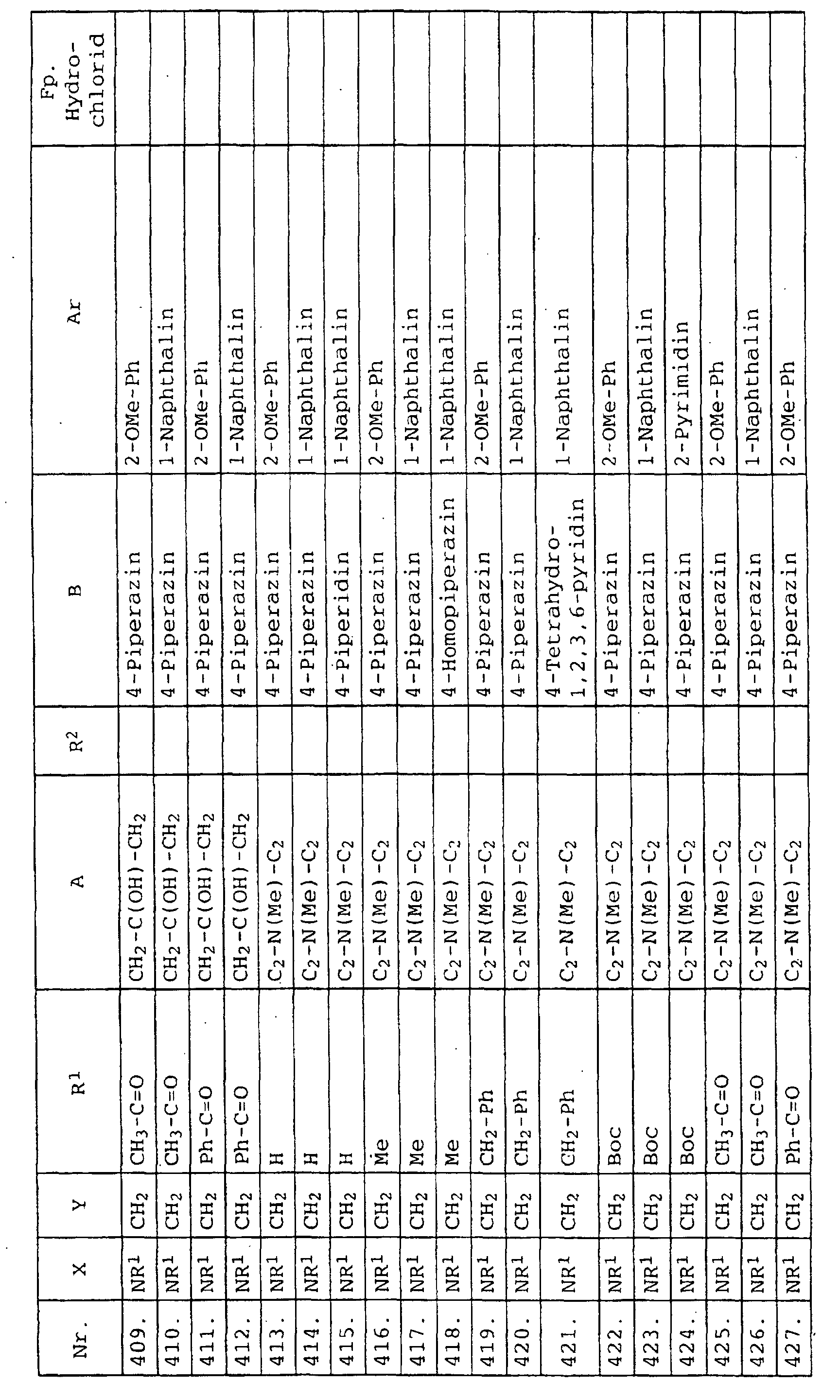

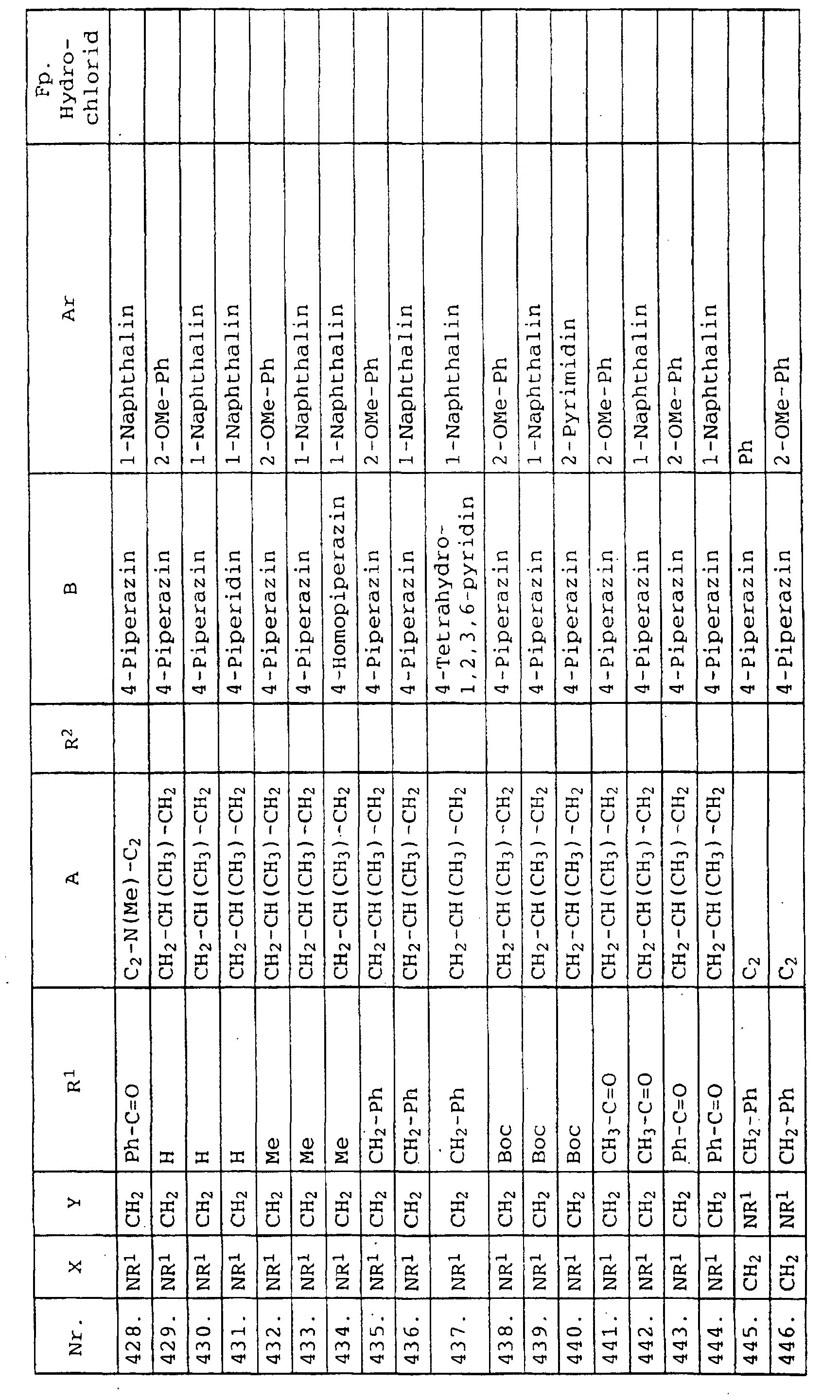

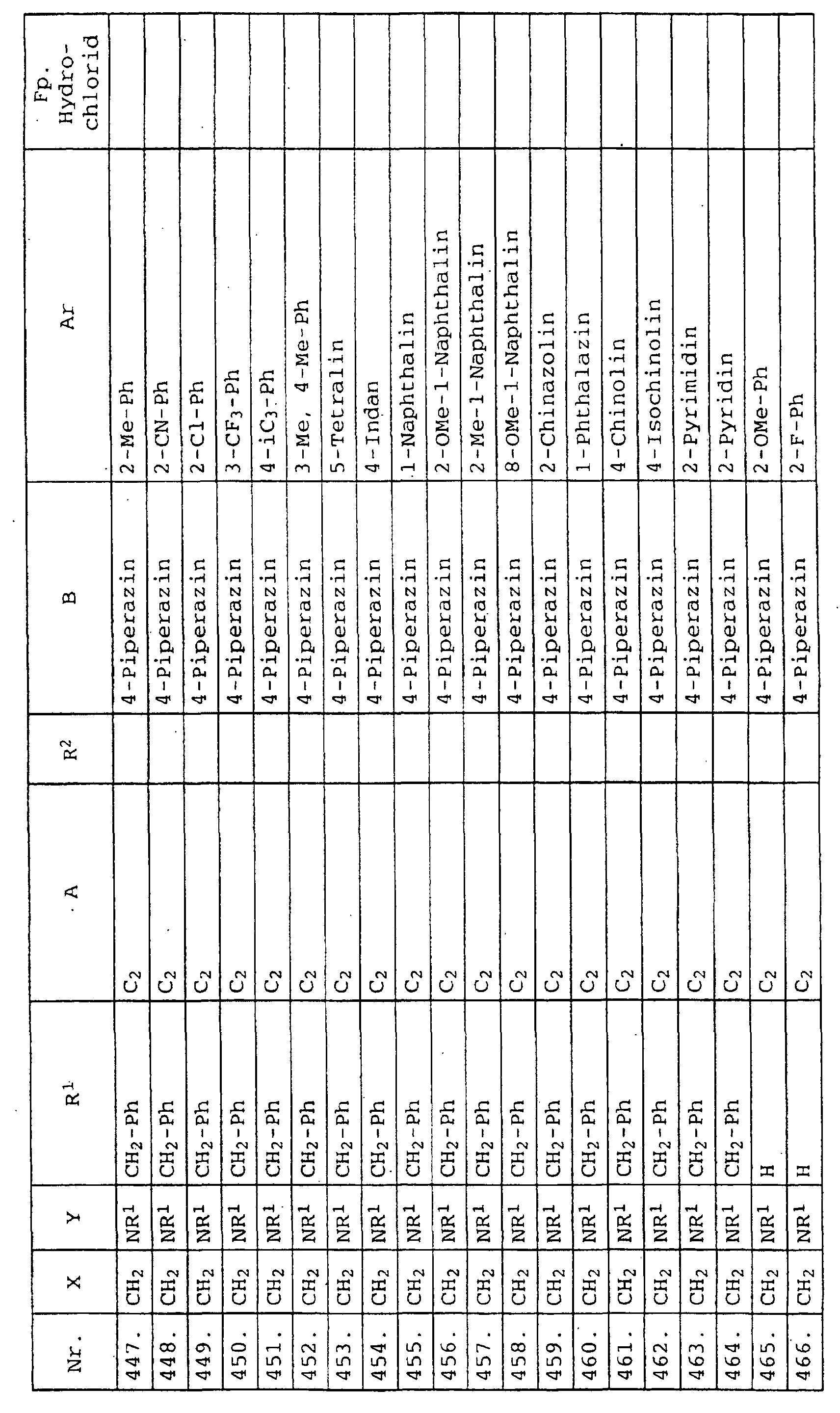

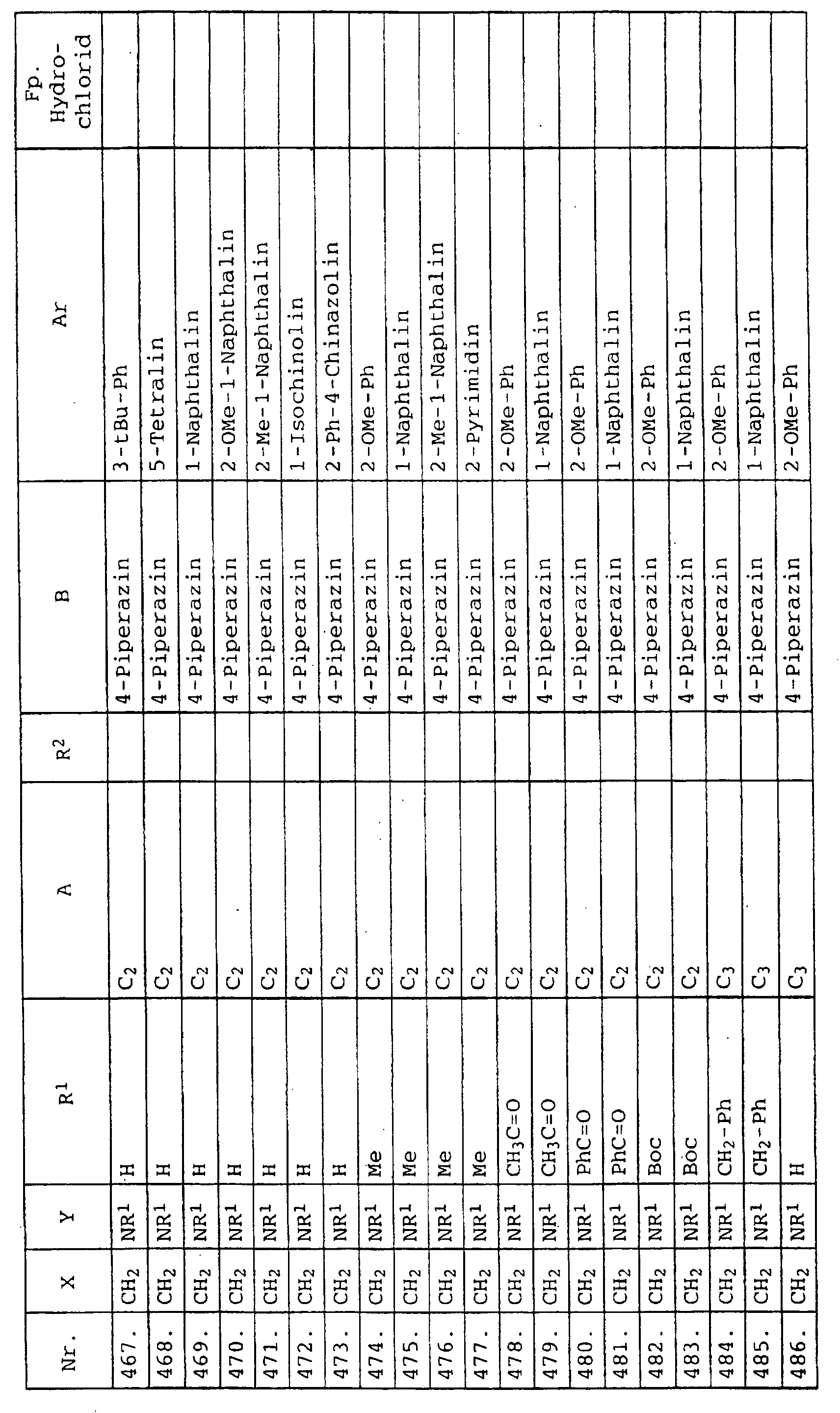

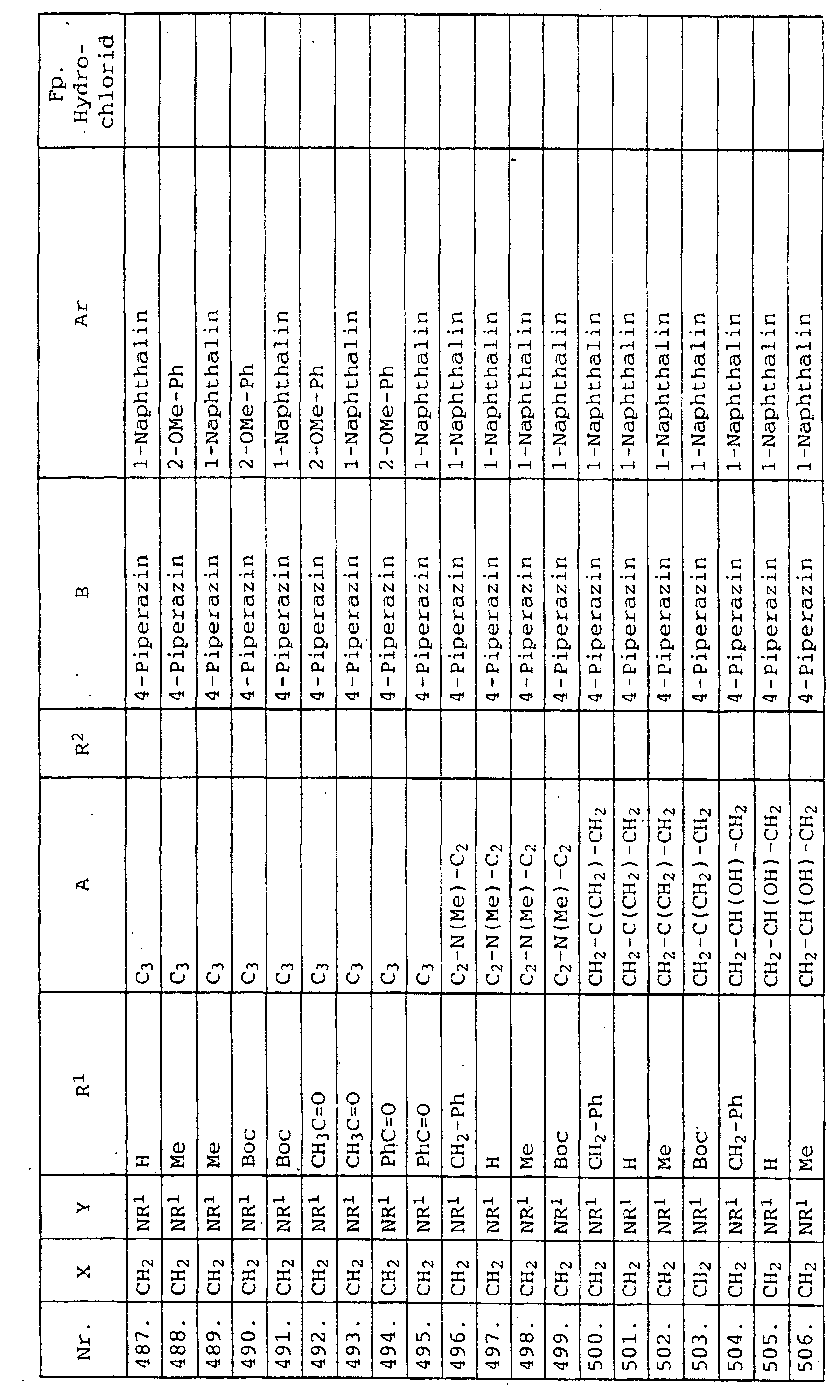

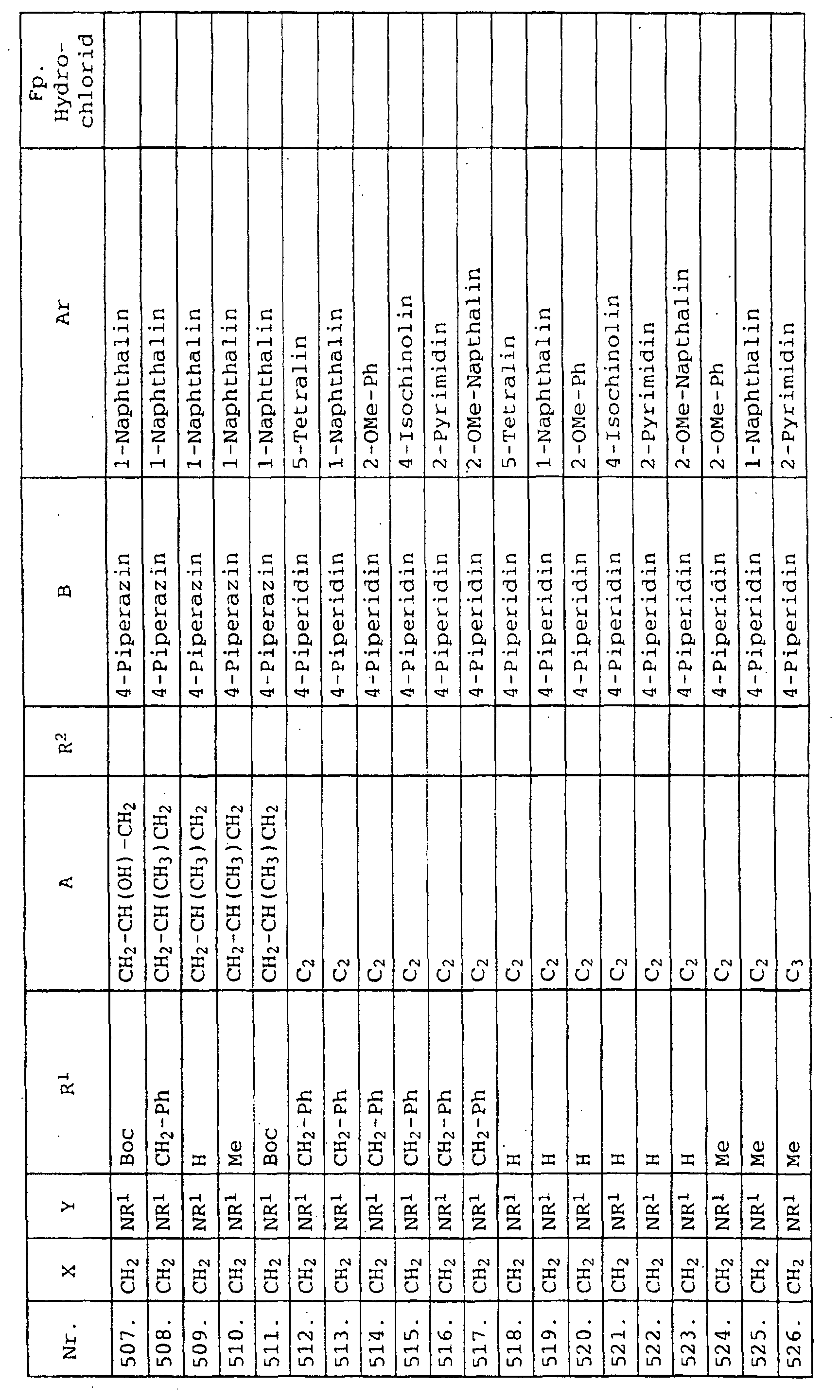

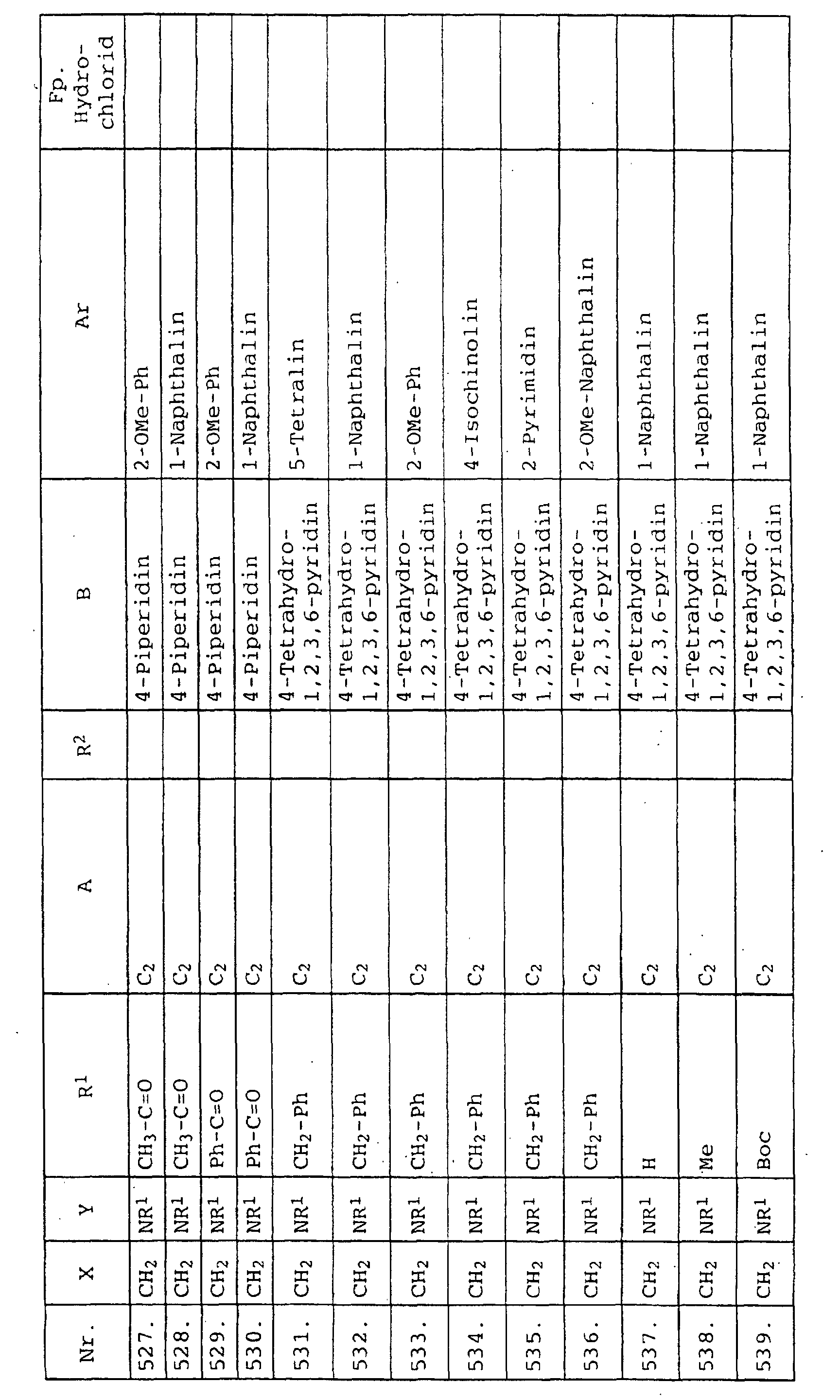

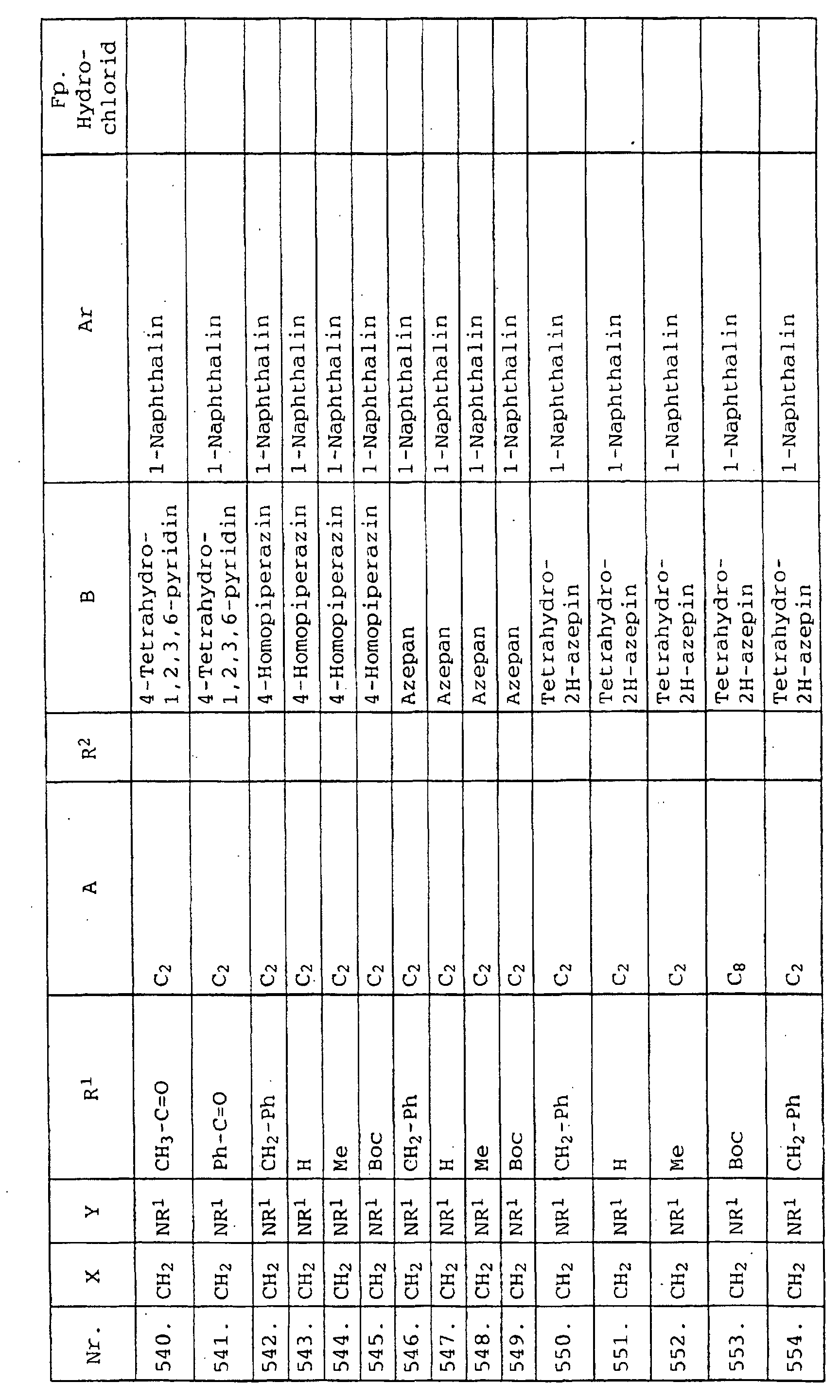

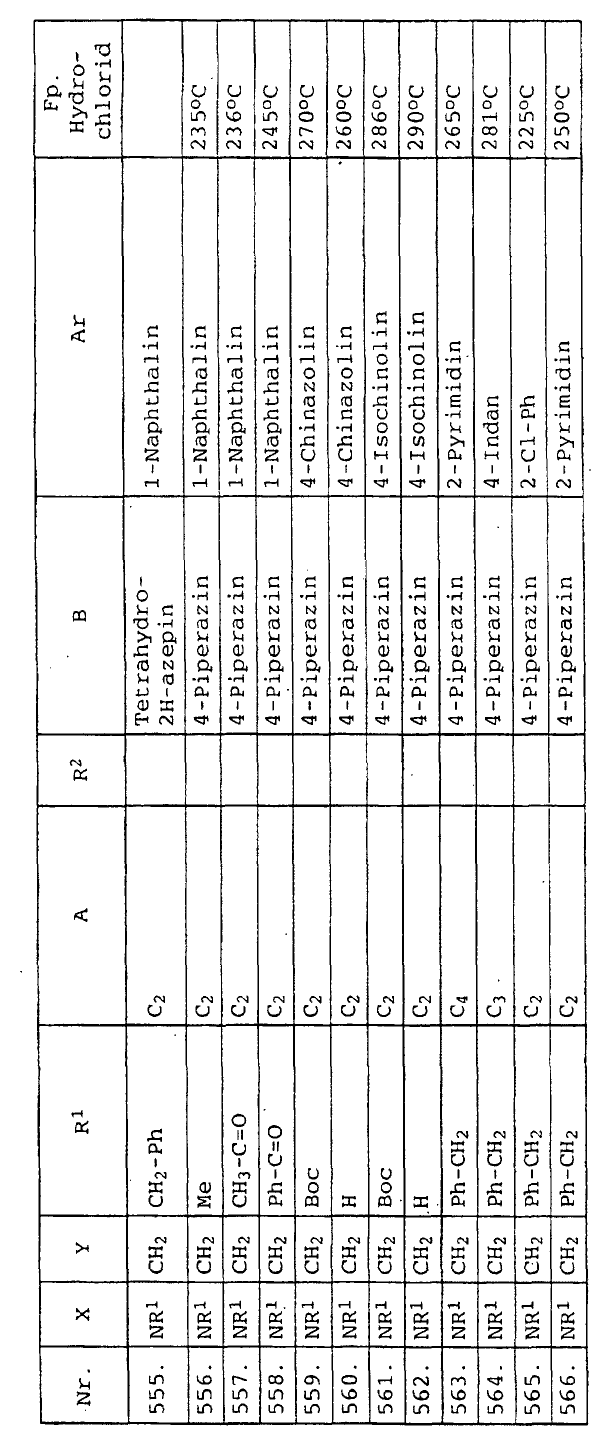

## Patentansprüche

1. 3-substituierte Tetrahydropyridopyrimidinon-Derivate der Formel I 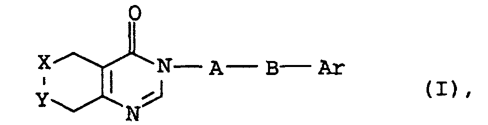 worin
einer der beiden Reste X, Y für CH₂ und der andere für NR¹ steht,
R¹ für Wasserstoff, (C₁₋₆) Alkyl verzweigt oder unverzweigt, CO-( C₁₋₄)-Alkyl, CO₂tBu, CO-Aryl und einen Phenylalkyl-C₁-C₄-Rest, der seinerseits am Aromaten durch F, Cl, Br, I, C₁-C₄ Alkyl, C₁-C₄ Alkoxy, Trifluormethyl, Hydroxy, Amino, Cyano oder Nitro substituiert sein kann, steht,
A für verzweigtes oder unverzweigtes (C₁₋₁₀)-Alkylen oder geradkettiges oder verzweigtes (C₂₋₁₀)-Alkylen steht, das wenigstens eine Gruppe Z umfaßt, die ausgewählt ist unter O, S, NR², Cyclopropyl, CO₂, CHOH, einer Doppel- oder einer Dreifachbindung,
R² für Wasserstoff und C₁-C₄ Alkyl steht,
B für 4-Piperidin, 4-Tetrahydro-1,2,3,6 pyridin, 4-Piperazin oder die entsprechenden um eine Methylengruppe vergrößerten Ringverbindungen steht, wobei die Verknüpfung zu A über ein N-Atom von B erfolgt und
Ar für Phenyl, das gegebenenfalls durch (C₁₋₆) Alkyl verzweigt oder unverzweigt, O-( C₁₋₆)-Alkyl verzweigt oder unverzweigt, OH, F, Cl, Br, I, Trifluormethyl, NR²₂, CO₂R², Cyano oder Phenyl substituiert ist, Tetralin, Indan, höherkondensierte Aromaten wie Naphthalin, das gegebenenfalls durch (C₁₋₄) Alkyl oder O(C₁₋₄) Alkyl substituiert ist, Anthracen oder 5- oder 6-gliedrige aromatische Heterocyclen mit 1 bis 2 Heteroatomen, die unabhängig voneinander ausgewählt sind unter O und N, die noch mit weiteren aromatischen Resten anelliert sein können,
steht sowie deren Salze mit physiologisch verträglichen Säuren.

2. 3-substituierte Tetrahydropyridopyrimidinon-Derivate nach Anspruch 1, wobei
einer der beiden Reste X, Y für CH₂ und der andere für NR¹ steht,
R¹ für Wasserstoff, (C₁₋₄) Alkyl verzweigt oder unverzweigt, CO-( C₁₋₄)-Alkyl, CO₂tBu, COPh oder einen Phenylalkyl C₁-C₂ Rest, der seinerseits am Aroinaten durch F, Cl, Br, I, C₁-C₄ Alkyl, C₁-C₄ Alkoxy, Trifluormethyl, Hycroxy oder Cyano substituiert sein kann, steht,
A für (C₂₋₅) Alkylen verzweigt oder unverzweigt oder eine (C₂₋₅) Alkylengruppe, die eine Gruppe Z umfaßt, die ausgewählt ist unter CHOH, Cyclopropyl, einer Doppel- oder einer Dreifachbindung,
B für 4-Piperidin, 4-Tetrahydro-1,2,3,6 pyridin, 4-Piperazin oder Homopiperazin steht, wobei die Verknüpfung zu A über ein N-Atom von B erfolgt und
Ar für Phenyl, das gegebenenfalls durch (C₁₋₆) Alkyl verzweigt oder unverzweigt, O-(C₁₋₆)-Alkyl verzweigt oder unverzweigt, F, Cl, Br, I, Trifluonmethyl, CO₂R², NR²₂, Cyano oder Phenyl substituiert ist, Tetralin, Indan, höherkondensierte Aromaten wie Naphthalin, das gegebenenfalls durch (C₁₋₄) Alkyl oder O(C₁₋₄) Alkyl substituiert ist oder 5- oder 6-gliedrige aromatische Heterocyclen mit 1 bis 2 Stickstoffen, die mit weiteren aromatischen Resten anelliert sein können, steht.

3. 3-substituierte Tetrahydropyridopyrimidinon-Derivate der Formel I nach Anspruch 1, wobei
einer der beiden Reste X, Y für CH₂ und der andere für NR¹ steht,
R¹ für Wasserstoff, (C₁₋₂) Alkyl, CO-( C₁₋₄)-Alkyl oder einen Phenylalkyl C₁-C₂ Rest steht,
A für (C₂₋₃) Alkyl steht,
B für 4-Piperidin, 4-Piperazin oder 4-Tetrahydro-1,2,3,6 pyridin steht und
Ar für Pyrimidin, Phenyl, das gegebenenfalls durch O(C₁₋₂) Alkyl in ortho-Position substituiert ist, Tetralin, Indan oder Naphthalin, das gegebenenfalls durch (C₁₋₄) Alkyl oder O(C₁₋₂) Alkyl substituiert ist, steht.

4. Verwendung von Verbindungen gemäß Anspruch 1-3 zur Herstellung von Arzneimitteln.

5. Verwendung nach Anspruch 4 zur Behandlung von Depressionen und verwandten Krankheiten.

6. Verwendung von Verbindungen gemäß Anspruch 1-3 zur Herstellung von selektiven 5HT_{1B-} und 5HT_{1A} - Antagonisten.

7. Verwendung nach Anspruch 6, wobei neben dem selektiven Serotonin-Antagonismus eine Hemmung des Serotonin Reuptake hinzukommt.

## Claims

1. A 3-substituted tetrahydropyridopyrimidinone derivative of the formula I 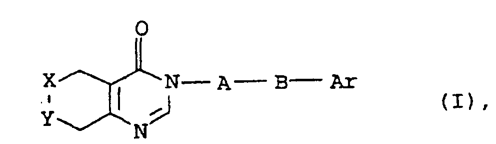 where
one of the two radicals X and Y is CH₂ and the other is NR¹,
R¹ is hydrogen, branched or unbranched (C₁-C₆) alkyl, CO-(C₁₋₄)-alkyl, CO₂tBu, CO-aryl or a phenyl-(C₁-C₄) alkyl radical which in turn may be substituted on the aromatic moiety by F, Cl, Br, I, C₁-C₄-alkyl, C₁-C₄ alkoxy, trifluoromethyl, hydroxyl, amino, cyano or nitro,
A is branched or unbranched (C₁-C₁₀)-alkylene or straight-chain or branched (C₂-C₁₀)-alkylene which contains at least one group Z which is selected from 0, S, NR², cyclopropyl, CO₂, CHOH or a double or triple bond,
R² is hydrogen or C₁-C₄ alkyl,
B is 1,4-piperidinylene, 1,2,3,6-tetrahydro-1,4-pyridinylene, 1,4-piperazinylene or the corresponding cyclic compounds enlarged by one methylene group, with the linkage to A being via an N atom of B, and
Ar is phenyl which is unsubstituted or substituted by branched or unbranched (C₁-C₆) alkyl, branched or unbranched 0-(C₁₋₆)-alkyl, OH, F, Cl, Br, I, trifluoromethyl, NR²₂, CO₂R², cyano or phenyl, or is tetralinyl, indanyl, a fused aromatic system such as naphthyl which is unsubstituted or substituted by (C₁-C₄) alkyl or 0(C₁₋₄) alkyl, anthryl or a 5- or 6-membered aromatic heterocycle having I or 2 heteroatoms which are selected, independently of one another, from 0 and N, which may be fused to further aromatic radicals,
or a salt thereof with a physiologically tolerated acid.

2. A 3-substituted tetrahydropyridopyrimidinone derivative as claimed in claim 1, where
one of the two radicals X and Y is CH₂ and the other is NR¹,
R¹ is hydrogen, branched or unbranched C₁-C₄ alkyl, CO-(C₁₋₄)-alkyl, CO₂tBu, COPh or a phenyl-(C₁-C₂) alkyl radical which in turn can be substituted on the aromatic moiety by F, Cl, Br, I, C₁-C₄ alkyl, C₁-C₄ alkoxy, trifluoromethyl, hydroxyl or cyano,
A is branched or unbranched (C₂₋₅) alkylene or (C₂-C₅) alkylene which contains a group Z which is selected from CHOH, cyclopropyl or a double or triple bond,
B is 1,4-piperidinylene, 1,2,3,6-tetrahydro- 1,4pyridinylene, 1, 4-piperazinylene or homopiperazinylene, where the linkage to A takes place via an N atom of B, and
Ar is phenyl which is unsubstituted or substituted by branched or unbranched (C₁₋₆) alkyl, branched or unbranched 0-(C₁₋₆) alkyl, F, Cl, Br, I, trifluoromethyl, CO₂R², NR²₂, cyano or phenyl, or is tetralinyl, indanyl, a fused aromatic system such as naphthyl which is unsubstituted or substituted by (C₁-C₄) alkyl or 0(C₁₋₄) alkyl, or a 5- or 6-membered aromatic heterocycle having 1 or 2 nitrogen atoms, which may be fused to other aromatic radicals.

3. A 3-substituted tetrahydropyridopyrimidinone derivative of the formula I as claimed in claim 1, where
one of the two radicals X and Y is CH₂ and the other is NR¹,
R¹ is hydrogen, C₁-C₂ alkyl, CO-(C₁₋₄)-alkyl or a phenyl (C₁-C₂) alkyl radical,
A is C₂-C₃ alkyl,
B is 1,4-piperidinylene, 1,4-piperazinylene or 1,2,3,6-tetrahydro-1,4-pyridinylene, and
Ar is pyrimidinyl, phenyl which is unsubstituted or substituted by 0(C₁₋₂) alkyl in the ortho position, tetralinyl, indanyl or naphthyl which is unsubstituted or substituted by C₁-C₄ alkyl or 0(C₁₋₂) alkyl.

4. The use of a compound as claimed in any of claims 1 to 3 for producing drugs.

5. The use as claimed in claim 4 for drugs for the treatment of depression and related disorders.

6. The use of a compound as claimed in any of claims 1 to 3 for producing selective 5-HT_{1B} and 5-HT_{1A} antagonists.

7. The use as claimed in claim 6, where the selective serotonin antagonism is supplemented by inhibition of serotonin reuptake.

## Revendications

1. Tétrahydropyridopyrimidinones substituées en position 3, répondant à la formule I 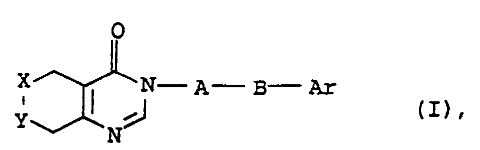 dans laquelle
l'un des deux symboles X et Y représente CH₂ et l'autre NR¹,
R¹ représente l'hydrogène, un groupe alkyle à chaîne droite ou ramifiée en C1-C6, un groupe CO-alkyle en C1-C4, CO₂tBu, CO-aryle ou phényl-alkyle en C1-C4 qui peut lui-même porter des substituants F, Cl, Br, I, alkyle en C1-C4, alcoxy en C1-C4, trifluorométhyle, hydroxy, amino, cyano ou nitro sur la partie aromatique,
A représente un groupe alkylène à chaîne droite ou ramifiée en C1-C10 ou un groupe alkylène à chaîne droite ou ramifiée en C2-C10 contenant au moins un chaînon Z choisi parmi O, S, NR², cyclopropyle, CO₂, CHOH, une double liaison ou une triple liaison,
R² représente l'hydrogène ou un groupe alkyle en C1-C4,
B représente un cycle 4-pipéridine, 4-tétrahydro-1,2,3,6-pyridine, 4-pipérazine ou un cycle correspondant agrandi d'un groupe méthylène, la liaison avec A se faisant par l'intermédiaire d'un atome d'azote de B et
Ar représente un groupe phényle portant éventuellement des substituants alkyle en C1-C6 à chaîne droite ou ramifiée, O-alkyle en C1-C6 à chaîne droite ou ramifiée, OH, F, Cl, Br, I, trifluorométhyle, NR²₂, CO₂R², cyano ou phényle, un cycle tétraline, indane, un cycle aromatique plus fortement condensé tel que naphtalène, portant éventuellement des substituants alkyle en C1-C4 ou O-alkyle en C1-C4, un cycle anthracène ou un hétérocycle aromatique à cinq ou six chaînons contenant un à deux hétéroatomes choisis, indépendamment l'un de l'autre, parmi O et N, et qui peut encore être condensé avec d'autres radicaux aromatiques,
et leurs sels d'acide acceptables pour l'usage pharmaceutique.

2. Tétrahydropyridopyrimidinones substituées en position 3, selon la revendication 1, pour lesquelles
l'un des deux symboles X et Y représente CH₂ et l'autre NR¹,
R¹ représente l'hydrogène, un groupe alkyle. à chaîne droite ou ramifiée en C1-C4, CO-alkyle en C1-C4, CO₂tBu, COPh ou phényl-alkyle en C1-C2, qui peut lui-même porter sur la partie aromatique des substituants F, Cl, Br, I, alkyle en C1-C4, alcoxy en C1-C4, trifluorométhyle, hydroxy ou cyano,
A représente un groupe alkylène à chaîne droite ou ramifiée en C2-C5 ou un groupe alkylène en C2-C5 comprenant un chaînon Z choisi parmi CHOH, cyclopropyle, une double liaison ou une triple liaison,
B représente un cycle 4-pipéridine, 4-tétrahydro-1,2,3,6-pyridine, 4-pipérazine ou homopipérazine, la liaison avec A se faisant par l'intermédiaire d'un atome d'azote de B et
Ar représente un groupe phényle portant éventuellement des substituants alkyle à chaîne droite ou ramifiée en C1-C6, O-alkyle en C1-C6 à chaîne droite ou ramifiée, F, Cl, Br, I, trifluorométhyle, CO₂R², NR²₂, cyano ou phényle, un cycle tétraline, indane, un cycle aromatique plus fortement condensé tel que naphtalène, portant éventuellement des substituants alkyle en C1-C4 ou O-alkyle en C1-C4, ou un hétérocycle aromatique à cinq ou six chaînons contenant un à deux atomes d'azote, et qui peut encore être condensé avec d'autres radicaux aromatiques.

3. Tétrahydropyridopyrimidinones substituées en position 3, répondant à la formule I de la revendication 1 dans laquelle
l'un des deux symboles X et Y représente CH₂ et l'autre NR¹,
R¹ représente l'hydrogène, un groupe alkyle en C1-C2, CO-alkyle en C1-C4 ou phényl-alkyle en C1-C2,
A représente un groupe alkyle en C2-C3,
B représente un cycle 4-pipéridine, 4-pipérazine ou 4-tétrahydro-1,2,3,6-pyridine et
Ar représente un cycle pyrimidine, un cycle phényle éventuellement substitué en position ortho par un groupe O-alkyle en C1-C2, un cycle tétraline, indane ou naphtalène éventuellement substitué par un groupe alkyle en C1-C4 ou O-alkyle en C1-C2.

4. Utilisation des composés selon les revendications 1 à 3 pour la préparation de médicaments.

5. Utilisation selon la revendication 4 pour le traitement des dépressions et des maladies apparentées.

6. Utilisation des composés selon les revendications 1 à 3 pour la préparation d'antagonistes sélectifs contre les récepteurs 5HT_{1B} et 5HT_{1A}.

7. Utilisation selon la revendication 6, dans laquelle, en plus de l'antagonisme sélectif contre la sérotonine, il y a inhibition d'une recapture de la sérotonine.
